(19) Europäisches Patentamt
European Patent Office
Office européen des brevets

(11) EP 2 911 574 B1

## (12) EUROPEAN PATENT SPECIFICATION

(45) Date of publication and mention
of the grant of the patent:
**13.07.2022  Bulletin 2022/28**

(21) Application number: **13786002.9**

(22) Date of filing: **14.10.2013**

(51) International Patent Classification (IPC):
*A61B 5/00* *(2006.01)*      *A61B 5/11* *(2006.01)*
*A61H 9/00* *(2006.01)*      *G16H 20/30* *(2018.01)*
*G16H 40/63* *(2018.01)*

(52) Cooperative Patent Classification (CPC):
**G16H 20/30; A61B 5/4848; A61B 5/4884;
A61H 9/0092; G16H 40/63;** A61B 5/1116;
A61B 5/1123; A61B 5/6804; A61B 5/6828;
A61H 2201/164; A61H 2201/5015;
A61H 2201/5043; A61H 2201/5048;
A61H 2201/5069; A61H 2201/5071;      (Cont.)

(86) International application number:
**PCT/US2013/064768**

(87) International publication number:
**WO 2014/066077 (01.05.2014 Gazette 2014/18)**

### (54) MONITORING SYSTEM FOR USE IN COMPRESSION THERAPY

ÜBERWACHUNGSSYSTEM ZUR VERWENDUNG BEI DER KOMPRESSIONSTHERAPIE

SYSTÈME DE SURVEILLANCE À UTILISER EN THÉRAPIE DE COMPRESSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.10.2012  GB 201219244**

(43) Date of publication of application:
**02.09.2015  Bulletin 2015/36**

(73) Proprietor: **3M Innovative Properties Company
St. Paul, MN 55133-3427 (US)**

(72) Inventors:
• **NEUENHAHN, Martin C.
41453 Neuss (DE)**
• **HITSCHMANN, Guido
41453 Neuss (DE)**
• **BICHEL, Jens
41453 Neuss (DE)**
• **NOWICKI, Anthony James
Woodbury, MN 55125 (US)**
• **KROPP, Karl Matthew
St. Paul, MN 55119-3279 (US)**

(74) Representative: **Mathys & Squire
Theatinerstraße 7
80333 München (DE)**

(56) References cited:
EP-A1- 2 436 349      US-A1- 2009 055 148
US-A1- 2011 015 498      US-A1- 2012 065 561

• HUGO PARTSCH: "The Static Stiffness Index: A Simple Method to Assess the Elastic Property of Compression Material In Vivo", DERMATOLOGIC SURGERY, vol. 31, no. 6, 1 June 2005 (2005-06-01), pages 625-630, XP055068602, ISSN: 1076-0512, DOI: 10.1111/j.1524-4725.2005.31604
• PARTSCH H ET AL: "Measurement of lower leg compression in vivo: Recommendations for the performance of measurements of interface pressure and stiffness", DERMATOLOGIC SURGERY, vol. 32, no. 2, February 2006 (2006-02), pages 224-233, XP002718927, ISSN: 1076-0512 cited in the application

• PARTSCH ET AL: "Interface pressure and stiffness of ready made compression stockings: Comparison of in vivo and in vitro measurements", JOURNAL OF VASCULAR SURGERY, vol. 44, no. 4, 1 October 2006 (2006-10-01), pages 809-814, XP005702698, C.V. MOSBY CO., ST. LOUIS, MO, US ISSN: 0741-5214, DOI: 10.1016/J.JVS.2006.06.024

(52) Cooperative Patent Classification (CPC): (Cont.)
A61H 2201/5082; A61H 2201/5084;
A61H 2201/5097; A61H 2205/106; A61H 2230/625

**Description**

Field

The present invention refers to a monitoring system for determining the efficacy of a compression device, which can be used in compression therapy, such as for treating chronic venous insufficiency. However, other fields of application are possible.

Background

In medical technology and medicine, a plurality of treatments of a human or animal body are known which imply the use of one or more compression devices for exerting pressure onto a body or a body part such as a limb of the human or animal user. Without restricting the present invention to a specific use, the treatment of venous diseases may be named, such as chronic venous insufficiency (CVI). In CVI, generally, veins are incapable of pumping a sufficient amount of oxygen-depleted blood to the heart. This disease, mostly, is closely related to thrombosis and, in many cases, implies an insufficient function of venous valves. Venous insufficiency generally may occur in a plurality of body parts such as limbs. Most frequently, legs or parts of the leg may be affected by venous insufficiency, such as calves.

As known in the art, venous insufficiencies and/or other types of diseases may be treated by compression therapy. Therein, a pressure is exerted onto the body part affected by the respective disease. As an example, compression bandages may be used, such as single layer or multi-layer compression bandages. A plurality of bandages is commercially available, mostly flexible bandages having a specific stiffness.

When using compression therapy, a number of precautions have to be taken in order to avoid injuries by overexerting pressure to the body part on the one hand side and exerting insufficient pressure on the other hand. Therefore, a plurality of devices is known in the art for monitoring pressure exerted onto the body part during compression therapy.

Document US2009055148 discloses a device that can be used as a tool for developing new ranges of orthoses, by simulating the action of a given restraint on the venous return of a typical population of patients and making it possible to evaluate the efficacy of a given restraint on the venous return, so as to define optimized pressure profiles.

In WO 2008/003920 A1, a patient compliance monitor for monitoring compliance of a patient to a treatment regime for treatment of a medical condition is disclosed. The compliance monitor comprises measurement means for measuring an external physical parameter acting on a limb of said patient, the external physical parameter having influence on the medical condition experienced by said limb. Inter alia, the use of a tilt sensor for measuring the tilt of a limb of said body, the use of a movement sensor for monitoring motion of said limb, the use of a pressure sensor for measuring the pressure applied to a region of said body, and the use of a thermometer for monitoring the ambient pressure around said body are disclosed. Further, recording means for recording data as well as comparative means for comparing the recorded data with data indicative of the treatment regime are disclosed, in order to determine patient compliance to the treatment regime.

US 2010/0010405 A1 discloses an apparatus and method for cyclically compressing the limb of a patient to improve blood flow in the limb. Inter alia, the use of a sensing device is disclosed which is capable of sensing a characteristic of a compression therapy performed by using the compression device.

In US 2011/0015498 A1, a system and a garment are disclosed which incorporate sensors that can be used for measuring or monitoring pressure or forces in feet, the stumps of limbs of an amputee that are fitted with prosthetic devices or any other parts of the body that are subject to forces when external pressure inducing devices are employed. Therein, one or more pressure sensors are integrally incorporated into a flexible substrate, fixed to the substrate or removably connected to the substrate.

Further, various monitoring systems are known which make use of a measurement of one or more key figures indicating patient compliance with compression therapy. As an example, US 2012/0083712 A1 discloses a monitoring system which is capable of monitoring venous refill time (VRT) via a pressure sensor in a bladder of a compression system. A controller of the compression system correlates the monitored VRT to a predetermined threshold to determine whether the patient is using the compression system.

In US 6,231,532 B1, a method for augmenting blood circulation in the limb of a patient is provided. Again, the venous refill time of the patient is measured. The limb is wrapped with a compression sleeve having at least one pressurizable chamber. The chamber is pressurized for a predetermined period of time to compress the limb and cause blood to flow out of the limb. The chamber is depressurized until the pressure in the chamber reaches a lower value, and the chamber is closed. The pressure in the chamber is sensed and the venous refill time is determined by sensing when the pressure reaches or will reach a plateau.

In US 7,127,370 B2, an attitude indicator device for detecting, indicating and/or logging the positional attitude of an individual in response to deviation from a set of one or more reference angles is disclosed. The device is mounted to the thigh of a patient and measurements are taken from an acceleration sensor within the device. The acceleration measurements are communicated to a receiver when the measurements deviate from acceptable thresholds, whereby the receiver indicates an alert condition.

Further, methods and devices are known which generally monitor the efficacy of compression therapy. Thus, in H. Partsch et al.: Measurement of lower leg compression in vivo: Recommendations for the performance of measurements of interface pressure and stiffness: A consensus statement, Dermatol Surg. 2006; 32: 224-233, general recommendations are provided for measuring the efficacy of compression systems by using one or more pressure sensors. Similarly, in G. Mosti et al: Comparison between a new, two-component compression system with zinc paste bandages for leg ulcer healing: a prospective, multicenter, randomized, controlled trial monitoring sub-bandage pressures, Wounds 2011; 23(5): 126-134, systems and methods for monitoring pressure exerted by compression systems are disclosed. Both documents provide an overview of different measurement techniques which may be used for determining exact pressures in compression therapy. Further, measurement routines implying resting and working pressure measurement on both legs are disclosed.

Summary of the invention

Despite the progress which has been made in compression therapy over the recent years, such as by the methods and devices disclosed in the above-mentioned documents, an ongoing need exists for devices and methods capable of effectively ensuring or assessing the efficacy of a compression device for use in compression therapy.

Specifically, this holds true with regard to precision and reproducibility of measurements, required for reliably providing current information on efficacy of compression therapy over time. Thus, specifically, due to changes and modifications in the materials of the compression systems over time and/or due to a curative effect of the compression therapy, the efficacy of the compression therapy may decrease over time, requiring attention by medical staff or the patient. Similarly, when initially applying a compression device, a precise and reliable online control is highly desirable allowing for preventing over-exerting pressure on the one hand side and providing an effective compression therapy on the other hand.

This need is fulfilled by a monitoring system for determining the efficacy of a compression device, having the features of the independent claim . Preferred embodiments of the invention, which may be realized in an isolated way or in an arbitrary combination, as the skilled person will recognize, are disclosed in the dependent claims.

As used in the following, the expressions "comprise", "include", "contain" or "have" as well as grammatical variations thereof are used in a non-exclusive way. Thus, the term "A comprises B" may refer both to the case in which A solely consists of B and to the case in which, besides B, A contains one or more further components or constituents.

In a first aspect of the present invention, a monitoring system for determining the efficacy of a compression device for use in compression therapy is disclosed. As used herein, the term efficacy may generally refer to an arbitrary parameter or combination of parameters indicative of the physical effect exerted by the compression device onto a body or body part of a user. As an example, the pressure exerted by the compression device may be a parameter or may be part of a set of parameters indicative of the efficacy.

As further used herein, the term compression therapy generally refers to an arbitrary type of therapy including exerting pressure onto a body or body part of a user such as to a limb of a user. As outlined above, compression therapy specifically may be used for curing chronic venous insufficiency (CVI) and/or any other type of disease related to CVI, such as chronic swelling of legs and ankles, ulcers and/or other diseases. However, other types of illnesses or injuries may be treated by using compression therapy, such as injuries induced by sports or accidents. Further, compression therapy may be used for preventive purposes, such as for preventing thrombosis. Thus, generally, compression therapy may be used for curative purposes as well as for preventive purposes.

As further used herein, the term compression device refers to an arbitrary device adapted for exerting pressure onto a body or body part of the user. The user, which may be a human or an animal, may also be referred to as a patient. However, the user not necessarily has to suffer from injuries and/or illnesses, since the invention may also be used for preventive purposes, such as for preventing thrombosis. The compression device, as will be outlined in further detail below, may preferably comprise one or more of a bandage, such as a flexible bandage, a sleeve, such as a flexible sleeve which may be put over a body part, specifically a limb, a garment capable of exerting pressure onto the body or a body part, or any other type of device capable of exerting pressure onto the body and/or the body part. Preferably, the compression device is capable of exerting pressure over an area of the body or body part which is at least 5 cm2, more preferably at least 50 cm$^2$, more preferably at least 100 cm$^2$ and, most preferably, at least 200 cm$^2$.

As further used herein, the term monitoring system generally refers to a one-component or multicomponent device capable of determining the efficacy once or several times, preferably repeatedly over a period of time.

The monitoring system comprises at least one pressure sensor for measuring a pressure exerted onto a body part of a user by the compression device. The monitoring system further comprises at least one attitude sensor for acquiring at least one attitude information on at least one of a position, an orientation and a movement of the user. The monitoring system further comprises at least one measuring device having at least one evaluation unit. The attitude information preferably may be an actual or current attitude information. The measuring device is adapted to communicate with the at least one pressure sensor and the at least one attitude sensor. The communication may fully or partially take place on a wire-basis and/or may fully or partially be a wireless communication. As outlined in further detail below, the at least one measuring device preferably is adapted to communicate with the at least one pressure sensor and/or the at least one attitude sensor wirelessly, such as by using RFID standard.

The evaluation unit is further adapted to receive at least one pressure value acquired by the pressure sensor. Further, the evaluation unit is adapted to receive at least one attitude information acquired by the attitude sensor. The at least one evaluation unit is adapted to automatically combine the at least one pressure value and the at least one attitude information in order to determine at least one key figure K indicating the efficacy of the compression device taking into account the attitude information.

The monitoring system is adapted for determining the efficacy of at least one compression device for use in compression therapy, wherein the efficacy of one or more compression devices may be determined.

As outlined above, the monitoring system comprises at least one pressure sensor for measuring a pressure exerted onto a body part of a user by the at least one compression device. As used herein, the term pressure sensor generally may refer to an arbitrary device capable of providing a signal and/or information indicative of the pressure exerted onto the body part by the compression device. Examples of pressure sensors capable of performing this type of measurement will be given in further detail below. For measuring the pressure exerted onto the body part, the pressure sensor may be located in between the compression device and the body part, such as in between the bandage and/or sleeve and the surface of the body part. Additionally or alternatively, the at least one pressure sensor may fully or partially be implemented into the compression device itself, such as by locating the pressure sensor in between several layers of the compression device, such as in between layers of a compression bandage. Again, additionally or alternatively, one or more additional layers may be interposed in between the pressure sensor and the skin of the patient, such as one or more layers of garment and/or one or more layers of tissue, which not necessarily have to be part of the compression device itself. Thus, by interposing one or more layers of tissue in between the compression device and the skin of the user, biocompatibility and/or comfort to the patient may be increased and/or the risk of inducing pain or even injuries may be reduced. Again, additionally or alternatively, the at least one pressure sensor may be located fully or partially outside the compression device. As an example, a bladder of the pressure sensor may be located underneath and/or within the compression device, and a tube or tube like device may fluidically transmit the pressure to a measuring part of the pressure sensor located outside the compression device.

The pressure sensor may be located in one or more positions or areas in which pressure information might be of interest to the user and/or to medical staff applying the therapy to the user. Thus, one or more positions of the pressure sensor may be chosen and/or pressure sensors extending over an extended area of the compression device may be used, such as pressure sensors extending over the whole length of the compression bandage. Various options are possible.

As further outlined above, the monitoring system comprises at least one attitude sensor for acquiring at least one attitude information on at least one of a position, an orientation and a movement of the user. Thus, as used herein, the term attitude may generally refer to a state of the user's body which may have an impact on compression therapy, such as by increasing or decreasing an internal pressure in the user's body or inside a body part of the user. Thus, the attitude may generally refer to one or more of a state of a position, an orientation and a movement of the full body or a body part of the user. Correspondingly, the term attitude information refers to an arbitrary information which is related to a attitude of the user, preferably a current attitude. The term attitude sensor refers to an arbitrary sensing device which, by itself or in combination with one or more other devices, is capable of providing the at least one attitude information and/or at least one measurement signal indicative of the attitude information.

As indicated above, the monitoring system further comprises at least one measuring device having at least one evaluation unit. As outlined in further detail below, the measuring device preferably may fully or partially be attached to the compression device or to the body of the user via the compression device, or alternatively it may be partially or fully be integrated into the compression device, or alternatively the measuring device may be simply, appropriately carried by the user. Favorably, the measuring device may have a weight of less than 1 kg, preferably of less than 500 g or even less than 200 g. In terms of volume, in order to be carried by the user, the measuring device preferably may have a volume of less than 500 cm$^3$, preferably of less than 200 cm$^3$ or even less than 100 cm$^3$. It will be appreciated that a low weight and/or small volume is desirable in terms of the user either carrying or wearing the measuring device.

The at least one measuring device is adapted to communicate, preferably wirelessly communicate, with the at least one pressure sensor and to receive at least one pressure value acquired by the at least one pressure sensor. Further, the at least one measuring device is adapted to communicate, preferably wirelessly, with the at least one attitude sensor and to receive at least one attitude information acquired by the at least one attitude sensor. The communication generally may be or may comprise a unidirectional communication and/or a bidirectional communication. The term wireless communication generally refers to a unidirectional or bidirectional communication via one or more of: an exchange of electromagnetic radiation, induction and electrostatic influence. The exchange of electromagnetic radiation preferably is an exchange of radio signals. Thus, as will be outlined in further detail below, a preferred way of wireless communication between the measuring device and the evaluation unit is a wireless communication according to the RFID standard. However, additionally or alternatively, other types of wireless communication are feasible.

Thus, the measuring device preferably may comprise a compact housing, such as a compact housing made of one or more of a plastic material, a metal and a ceramic material. If desired, for the comfort of the user, the housing may be covered with a foam or another type of soft material. Inside the compact housing, the evaluation unit as well as, optionally, further elements of the measuring device may be located. Further, the measuring device may favorably comprise at least one interface. The measuring device may provide one or more unidirectional and/or bidirectional user interfaces, such as for allowing for the user to operate the measuring device in order to provide commands and/or provide information to the measuring device and/or for allowing for the measuring device to provide information to the user, such as one or more of visual information, acoustic information and tactile information. Additionally or alternatively, the measuring device may have one or more electronic interfaces for unidirectional or bidirectional exchange of commands and/or information with one or more other devices. As will be outlined in further detail below, the measuring device preferably may have one or more radio frequency (RF) and/or infrared interfaces, specifically for communicating with one or more display and control devices, such as one or more mobile communication devices like hand-held phones and/or smartphones and/or tablet PCs.

With regard to the setup of the at least one measuring device, the at least one pressure sensor and the at least one attitude sensor, various embodiments are feasible. Thus, the at least one pressure sensor may be located outside the at least one measuring device, such as spatially separated from the at least one measuring device. In case a plurality of pressure sensors is used, one of these pressure sensors, a plurality of these pressure sensors or even all of these pressure sensors may be located outside the at least one measuring device, such as spatially separated from the at least one measuring device. Additionally or alternatively, the at least one pressure sensor may fully or partially be integrated into the at least one measuring device. Thus, the at least one pressure sensor may fully or in part be integrated into a housing of the measuring device. As an example, a sensing portion, such as a fluid-filled (e.g. a gas and/or liquid-filled) bladder of the pressure sensor may be located outside the measuring device, whereas a measurement portion of the pressure sensor may be located inside the measuring device, wherein the bladder and the measurement portion may be connected via at least one tube. Other embodiments are feasible. In case a plurality of pressure sensors is provided, one of these pressure sensors, a plurality of the pressure sensors or even all of these pressure sensors may be integrated into the measuring device. Further, at least one pressure sensor may be fully or partially integrated into the measuring device, whereas at least one pressure sensor may be located spatially separated from the measurement device.

Similarly, with regard to the at least one attitude sensor, the at least one attitude sensor may be located outside the at least one measuring device, such as spatially separated from the at least one measuring device. In case a plurality of attitude sensors is used, one of these attitude sensors, a plurality of the attitude sensors or even all of these attitude sensors may be located outside the at least one measuring device, such as spatially separated from the at least one measuring device. As an example, the at least one attitude sensor and/or at least one of a plurality of attitude sensors may be located at a different body part of the user, separate from the body part onto which the pressure is exerted by the compression device. Thus, as an example, the compression device may act onto a calf of the user, wherein at least one attitude sensor is located on a thigh of the user. Additionally, at least one attitude sensor may be located on the calf of the user. Various embodiments are feasible and will be disclosed in further detail below. Again, additionally or alternatively, the at least one attitude sensor may fully or partially be integrated into the at least one measuring device. Thus, the at least one attitude sensor may fully or in part be integrated into a housing of the measuring device. In case a plurality of attitude sensors is provided, one of these attitude sensors, a plurality of the attitude sensors or even all of these attitude sensors may be integrated into the measuring device. Further, at least one attitude sensor may be fully or partially integrated into the measuring device, whereas at least one attitude sensor may be located spatially separated from the measurement device.

As will be outlined in further detail below, the measuring device preferably may be attached to the compression device and/or integrated into the compression device. Thus, the measuring device may be adapted to be integrated into the compression device and/or attached to the compression device, preferably on an outer side of the compression device. As an example, the measuring device may comprise one or more attachment elements for attaching the measuring device to the compression device, such as one or more hooks and/or one or more Velcro fasteners. Preferably, this attachment and/or integration is such that the measuring device may still be actuated by the user and/or may still provide one or more signals to the user, such as visual and/or acoustic and/or tactile signals. Thus, preferably, the measuring device may be attached to the compression device and/or may be integrated into the compression device such that at least one surface of the measuring device is accessible to the user from the outside.

The term evaluation unit, as used herein, generally refers to an arbitrary device or combination of devices capable of evaluating one or more signals provided by the pressure sensor. The signals provided by the pressure sensor may be or may comprise one or more electronic signals. The evaluation unit may comprise one or more data processing devices, such as one or more processors, specifically one or more microprocessors, and/or one or more integrated circuits, such as one or more application-specific integrated circuits (ASICs). Additionally, the evaluation unit may comprise one or more data storage devices, such as one or more volatile and/or non-volatile data storage devices.

The at least one evaluation unit may be integrated into a compact housing of the measuring device.

The evaluation unit is adapted to receive at least one pressure value acquired by the at least one pressure sensor and at least one attitude information acquired by the attitude sensor. Thus, the evaluation unit may receive one or more electronic measurement signals indicative of the pressure value and/or indicative of the attitude information. Therein, the at least one pressure value acquired by the at least one pressure sensor and/or the at least one attitude information acquired by the at least one attitude sensor may be used by the evaluation unit as "raw" data, i.e. without any modifications. However, additionally or alternatively, the evaluation unit may also be adapted to perform one or more preprocessing operations on the at least one pressure value and/or on the at least one attitude information, such as at least one filtering and/or averaging operation. Thereby, preprocessed data comprising the at least one pressure value and/or preprocessed data comprising the at least one attitude information may be generated. Thus, as will be outlined in further detail below, an averaging over a predetermined number of neighboring values, such as over 10 neighboring values, may be performed, in order to obtain smoothened measurement curves. In the following, no difference will be made between the use of raw data and the use of preprocessed data for determining the at least one key figure, since both options are possible.

The evaluation unit may directly or indirectly receive the pressure value and/or the attitude information. Thus, a direct unidirectional or bidirectional exchange of information may take place between the evaluation unit and the pressure sensor and/or the attitude sensor. Additionally or alternatively, one or more communication components may be inserted in between the evaluation unit and the pressure sensor and/or the attitude sensor. Thus, the measuring device may comprise one or more communication components for unidirectionally and/or bidirectionally exchanging information and/or commands with the at least one pressure sensor and/or the attitude sensor, preferably via RFID. The evaluation unit may process the at least one pressure value and/or the at least one attitude information and/or may store the at least one pressure value and/or the at least one attitude information in one or more data storage devices.

The evaluation unit is adapted to automatically combine the pressure value and the attitude information in order to determine at least one key figure K indicating the efficacy of the compression device taking into account the attitude information. For this purpose, the evaluation unit may comprise at least one hardware and/or software, such as at least one computer program, in order to perform at least one algorithm for determining the at least one key figure K indicative of the efficacy of the compression device, by processing the attitude information and the pressure value. As will be outlined in further detail below, the term key figure generally refers to an arbitrary measure of the efficacy of the compression device. Examples of key figures will be given in further detail below.

The monitoring system may further comprise at least one display and control device, wherein the display and control device is adapted to communicate with the measuring device. The communication may fully or partially be a wireless communication. Additionally or alternatively, a wire-based communication may be used. Preferably, the display and control device is adapted to wirelessly communicate with the measuring device. As used herein, the term display and control device may generally refer to an arbitrary device which is capable of providing commands to the measuring device, such as for starting a measurement and/or for requesting measurement values, such as pressure values and/or attitude information. Additionally or alternatively, the display and control device may refer to an arbitrary device which is capable of receiving information from the measuring device and directly or indirectly displaying the information to a user. Thus, the display and control device may be adapted to receive information from the measuring device, such as information relating to the at least one pressure value and/or the at least one attitude information. The display and control device may directly display this information to the user and/or may display the information to the user after one or more steps of processing the information. Thus, the display and control device may comprise one or more processors which are adapted by appropriate computer programs to further evaluate the information provided by the measuring device.

The display and control device preferably may be a device separate from the measuring device. Thus, the measuring device may be attached to the compression device and/or may be integrated into the compression device and/or may be worn by the user, whereas, the display and control device may be favorably handled independently, such as manually by the user. Preferably, the display and control device may be a hand-held device. Thus, the display and control device preferably may be a compact device having a weight of preferably less than 1 kg, more preferably of less than 500 g or even less than 300 g. Preferably, the display and control device may have a volume of less than 1000 cm$^3$, more preferably of less than 500 cm$^3$ or even less than 300 cm$^3$. The display and control device preferably may have a display, such as a matrix display, for displaying information to the user. The information may displayed numerically or through by other indicia, such as colors (e.g. the color green for indicating the system is good, yellow for indicating a warning or red for indicating an potential problem).

As a preferred option, the display and control device may be a standard device which may serve one or more additional purposes than the purpose of monitoring the efficacy of the compression device. Thus, preferably, the display and control device may be a mobile communication device, preferably a hand-held phone and/or a smartphone.

As outlined above, the display and control device is adapted to communicate with the measuring device, preferably wirelessly. The wireless communication preferably may take place via electromagnetic radiation, such as via infrared radiation and/or radiofrequency radiation. Thus, preferably, the display and control device may be adapted to communicate with the measuring device via one or more of Bluetooth, infrared and radio data transmission. However, as outlined above, alternatively or additionally, a wire-based communication between the display and control device and the measuring device may be used, such as a communication by using USB standard.

Further preferred embodiments refer to the communication between the measuring device and the pressure sensor, which, preferably, is a wireless communication. As outlined above, this communication may be a unidirectional communication, in an arbitrary direction, and/or a bidirectional communication. Most preferably, as outlined above, the communication takes place via RFID. Thus, preferably, the measuring device may be adapted to communicate with the pressure sensor via RFID, preferably according to ISO/IEC standard 15693-3. Therein, the RFID communication may also be used for providing energy to the pressure sensor. Thus, as an example, energy may be supplied to the pressure sensor by the measuring device, preferably in a wireless fashion. Consequently, the pressure sensor preferably may be a passive pressure sensor without any battery of its own and without any accumulator of its own. The measuring device, on the other hand, preferably may comprise at least one electric energy storage, such as at least one battery and/or at least one accumulator.

In case the measuring device is adapted to communicate via RFID, the wireless RFID communication may also be used for communicating with one or more additional sensors, such as with the at least one attitude sensor and/or with one or more optional temperature sensors. Similarly to the communication with the pressure sensor, the measuring device may also provide energy to the one or more additional sensors, such as to the at least one attitude sensor and/or to the one or more optional temperature sensors.

As indicated above, the monitoring system, besides the at least one pressure sensor and the at least one attitude sensor, may further comprise one or more further sensors. These sensors may be placed in various positions, such as underneath all within the compression device and/or in other positions, such as on a body surface of the user. Further, the one or more sensors may fully or partially be integrated into the measuring device. Thus, as an example, the monitoring system may comprise one or more temperature sensors. In this case, preferably, the evaluation unit may be adapted to correct the at least one pressure value for temperature-dependent influence. Additionally or alternatively, the evaluation unit may be adapted to take into account the temperature provided by the at least one temperature sensor when determining the at least one key figure K indicating the efficacy of the compression device. Thus, and increased blood pressure due to high temperature may be corrected for. Additionally or alternatively, a known temperature-induced change in elastic properties or stiffness of the compression device may be corrected for. Further, additionally or alternatively, a known temperature dependency of the pressure sensor may be corrected for.

The monitoring system may further be adapted to perform one or more operations by using the at least one pressure value and/or the at least one attitude information. Generally, the attitude information may be stored to provide an activity profile or a part of an activity profile of the user over a time span. Thereby, by tracking the attitude information over a specific time span, specific developments in the attitude information may be determined and evaluated.

As an example, the evaluation unit generally may be adapted to automatically determine if the user is sleeping. Thus, in case no change in the attitude information over a predetermined time span is detected or in case the attitude information only changes by an insignificant amount over a specific time span, the evaluation unit generally may determine that the user is either sleeping or resting. Additionally or alternatively, the evaluation unit may be adapted to determine if the user is sleeping in case a horizontal orientation of the user and a standstill of the user are detected. Additional parameters may be used for determining if the person is sleeping, such as a body temperature and/or a blood pressure.

In case the evaluation unit determines that the user is sleeping, the evaluation unit may automatically switch into a sleep mode. Thus, the sleep mode may imply a reduced consumption of energy for the evaluation unit and/or other parts of the monitoring system, such as by a reduced rate of measurement. Thus, the sleep mode may imply a reduced frequency of acquisition of pressure values and attitude information. The evaluation unit may be adapted to switch back into a normal mode in case a rising of the user is detected. Thus, the evaluation unit may be adapted to detect the rising of the user via a signal change in at least one signal provided by at least one of an acceleration sensor and an orientation sensor.

The evaluation unit may further be adapted to detect other types of attitudes and/or activities, in addition or alternatively to a sleeping. Thus, the evaluation unit may be adapted to determine if the user is walking. The evaluation unit may be adapted to determine if the user is walking by identifying regular changes in at least one measurement curve. As an example, the measurement curve may be a measurement curve of pressure values, since a walking motion typically implies regular changes in pressure. Additionally or alternatively, the measurement curve may be or may imply a measurement curve of motion values, such as a measurement curve of acceleration values acquired by at least one motion sensor and/or acceleration sensor, since a walking motion typically implies regular changes in acceleration.

As discussed above, the attitude may comprise one or more states of the user. Thus, the attitude may refer to the whole body of the user and/or to a specific body part or combination of body parts of the user. For determining the attitude and/or for acquiring the at least one attitude information, one or more attitude sensors may be used. As an example, the attitude sensor may comprise at least one orientation sensor. Various types of orientation sensors are known in the art. As an example, the orientation sensor may comprise at least one of a gyroscope, an inclinometer, an angulation sensor and a tilt sensor. Additionally or alternatively, the attitude sensor may comprise at least one acceleration sensor. Again, additionally or alternatively, the at least one attitude sensor may comprise one or more altitude sensors and/or one or more magnetic field sensors. Generally and most preferably, the attitude sensor may comprises at least one micromechanical attitude sensor.

Further preferred embodiments refer to the measuring device. As discussed above, the monitoring device may comprise one or more user interfaces, which may be unidirectional and/or bidirectional. As an example, the measuring device may at least one display device. The display device may comprise at least one segmented display device and/or alphanumeric a display device, such as a passive matrix display and/or active-matrix display, in order to provide specific information or general indicia to the user. Additionally or alternatively, the display device may comprise one or more other types of display devices, such as one or more of an optical, acoustic and tactile indicator device.

Specifically in case the measuring device comprises one or more display devices, the evaluation unit may be adapted to generate a warning output via the indicator device in case one or more critical situations are recognized. Specifically, the evaluation unit may be adapted to generate the warning output in case one or more of the following situations are recognized:

- the compression device is found to be ineffective;
- the compression device is found to exert an overpressure;
- an external overpressure is found to act onto the compression device.

Herein, the term "ineffective" may refer to the fact that one or more key parameters as determined by the monitoring system are found to be out of range, such as below or above one or more predetermined efficacy thresholds.

Further, additionally or alternatively, the evaluation unit may be adapted to generate an instruction output via the indicator device in case a specific user action is found to be required. As an example, in case an inefficacy of the compression device should be recognized, the evaluation unit may be adapted to generate instructions to the user to change the compression device and/or to modify the compression device in order to restore efficacy. Additionally or alternatively, in case an overpressure should be detected, instructions may be given to loosen the compression device and to reduce the pressure exerted onto the body or body part of the user.

The evaluation unit is adapted to perform a real-time determination of the key figure. As used herein, the term real-time may refer to the fact that the determination of the key figure takes place immediately after acquiring the at least one pressure value and/or the at least one attitude information. Preferably, the determination of the key figure takes place within a time span of no more than 60 seconds after the acquisition of the pressure value and the attitude information, more preferably within a time span of no more than 30 seconds. In case a plurality of pressure values and/or a plurality of units of attitude information is acquired, the above-mentioned time spans may start when the last pressure value and/or the last attitude information is acquired.

As outlined above, the monitoring system may comprise one or more additional sensors besides the above-mentioned at least one pressure sensor and the at least one attitude sensor. Thus, as an example, the monitoring system additionally may comprise at least one ambient pressure sensor, wherein the at least one ambient pressure sensor is adapted to determine at least one ambient pressure acting onto at least one of the compression device and the body part from an outer side of the compression device. Thus, the at least one ambient pressure sensor may be adapted to determine an additional force and/or pressure exerted onto the compression device and/or the body part from the outside, such as by the users own weight resting on a support. Consequently, the ambient pressure may be a pressure exerted onto at least one of the compression device and the body part due to the user resting on a support, thereby exerting pressure onto the compression device due to a body weight of the user.

The monitoring system may comprise one or more attitude sensors. Preferably, the monitoring system may comprise a plurality of attitude sensors to be located in different regions of the body of the user. The evaluation unit may be adapted to automatically determine a attitude of the user by combining attitude information from the plurality of attitude sensors. Examples of various types of combinations of attitude information from different body parts for determining a attitude of the user will be given in further detail below.

As an example, the plurality of attitude sensors may comprise at least one thigh orientation sensor and at least one calf orientation sensor. This is due to the fact that many attitudes of the user, such as a resting position and a standing position, may be distinguished by using a combination of a thigh orientation sensor and a calf orientation sensor. Thus, the evaluation unit may be adapted to automatically determine if the user is in an upright position when both the thigh orientation sensor and the calf orientation sensor indicate a substantially vertical orientation.

As used herein and as will be used in the following, when referring to orientations, the term substantially refers to the fact that, preferably, precisely the named orientation is present. However, the term substantially may include tolerances with regard to the orientation, such as tolerances of no more than 30°, preferably of no more than 20°. Consequently, a substantially vertical orientation refers to a vertical orientation, wherein deviations of no more than 30° from the vertical orientation may be tolerated.

The monitoring system generally may further comprise at least one motion sensor. In combination with the above-mentioned at least one optional thigh orientation sensor and the at least one optional calf orientation sensor, the evaluation unit may be adapted to automatically determine if the user is in a standing position when an upright position is determined and the motion sensor indicates a standstill.

As outlined above, the monitoring system may further comprise one or more additional sensors. In a preferred embodiment, the monitoring system may further comprises at least one foot pressure sensor which is adapted to be positioned underneath a foot of the user and to acquire a force exerted by a weight of the user. The foot pressure sensor may also count as an attitude sensor as defined above, since the foot pressure sensor by itself or in combination with one or more other attitude sensors may allow for determining a user's attitude.

Precisely one foot pressure sensor may be provided, to be placed underneath one foot. Preferably, however, at least two foot pressure sensors are provided, wherein at least one of these foot pressure sensors is to be placed underneath each foot. An information provided by the at least one foot pressure sensor may be evaluated by the evaluation unit in order to determine the attitude of the user. Thus, a standing position and/or a walking position may be detected in case a high pressure signal or a high force signal is generated by the foot pressure sensor. Further, in case at least one foot pressure sensor is provided underneath each foot, the evaluation unit may be adapted to compare the signals provided by the foot pressure sensors. Thus, by detecting periodic alterations in the signals and, optionally, phase shifts in the periodic alterations of the signals of the at least two foot pressure sensors, a walking motion may be detected. Further, additionally or alternatively, the at least one foot pressure signal provided by the at least one foot pressure sensor may also be combined by the evaluation unit with at least one further information and/or signal provided by at least one further sensor. Thus, the user's attitude may be determined more precisely by combining the foot pressure signal with at least one orientation signal provided by at least one orientation sensor and/or with at least one further sensor signal provided by at least one further attitude sensor. Examples will be given in further detail below.

The monitoring system may further comprise at least one motion sensor. Again, the motion sensor may count as the attitude sensor and/or may be one of a plurality of attitude sensors. The motion sensor may be adapted to acquire at least one information regarding a motion of the user or of a body part of the user. Thus, the motion sensor may comprise one or more acceleration sensors. As outlined above, the evaluation unit may be adapted to determine an end of a sleeping phase of the user in case the motion sensor detects a high acceleration. Additionally or alternatively, the evaluation unit may be adapted to recognize certain movements of the user, such as a walking movement in case a periodic alteration in a signal of the motion sensor is detected. Again, the evaluation unit may be adapted to evaluate the signal of the motion sensor by itself and/or in combination with any other sensor signal, in order to determine the users attitude. Thus, a plurality of motion sensors may be provided, such as one motion sensor for each leg. In the latter case, a walking motion may be detected in case the motion sensors provide periodic signals having a phase shift. Further examples will be given below.

The evaluation unit may further be adapted to acquire at least one resting pressure $p_{rest}$, with the user being in a resting position. As used herein, the term resting position generally refers to an arbitrary, nonupright position, in which the user may fully or partially relax, specifically in a state in which muscles of the body part to which the compression therapy is applied are relaxed. As outlined in further detail below, the resting position preferably may be a supine position, in which the user sits on a couch or lounger, with his legs in a relaxed, flexed position. For the purpose of acquiring the at least one resting pressure, the evaluation unit may provide an appropriate processor and, preferably, an appropriate software for performing a measurement routine for acquiring at least one information indicating the at least one resting pressure $p_{rest}$.

The evaluation unit is adapted to determine at least one extended standing pressure $p_{standing, extended}$. Again, the evaluation unit provides an appropriate measurement routine, such as by providing an appropriate software capable of running on the processor, adapted for determining the at least one extended standing pressure.

As used herein, the term extended standing pressure refers to a pressure acquired with the user being in a standing position, which is acquired to the following procedure deviating from conventional measurements of the standing pressure $p_{standing}$. As used herein, the term standing position refers to an upright position of the user, wherein the user preferably equally weights down on both legs.

As opposed to the standing pressure $p_{standing}$, which usually is measured by simply measuring the pressure at a predetermined point in time after bringing the user into the standing position, the extended standing pressure is acquired by using the following procedure:

- the evaluation unit acquires a measurement curve of pressure values after a position change of the user into the standing position; and

- a slope of the measurement curve is automatically compared to at least one endpoint threshold value and, depending on a result of the comparison, an endpoint of a change in the measurement curve induced by the position change is automatically detected and a pressure value acquired at or after the endpoint is assigned to the extended standing pressure $p_{standing}$, extended.

As used herein, the term pressure value refers to an arbitrary item or amount of information indicating a specific pressure at a specific measurement time. The term measurement curve refers to a plurality of pressure values acquired at different points in time, wherein the measurement curve additionally may comprise the measurement times of the pressure values, such as by comprising value pairs of measurements times and corresponding pressure values acquired at the specific measurement times. As outlined in further detail below, the system and the method may make use of a plurality of measurement curves, which may be identical or non-identical. Thereof, at least one measurement curve may be used for determining the extended standing pressure. Further, as discussed above, the measurement curve may be subject to one or more filtering and/or averaging algorithms before making further use of the measurement curve, such as by averaging over a plurality of values of the measurement curve, such as 10 neighboring values of the measurement curve. In the following, no difference will be made between the use of the "raw" measurement curve, i.e. the use of the measurement curve without applying an averaging and/or filtering algorithm, and a measurement curve after applying an averaging and/or filtering algorithm, since both options are possible.

The acquisition of the measurement curve used for determining the extended standing pressure may start before, during or after the position change of the user into the standing position. The position change may take place from a generally arbitrary position being different from the standing position into the standing position, such as from a resting position into the standing position.

As further used herein, the term slope of the measurement curve generally refers to a curve indicating the increase or decrease over time of the measurement curve. Again, this curve may be subject to an averaging and/or filtering algorithm, such as by averaging over a plurality of values of the curve, such as over 10 neighboring values. In the following, no difference will be made between the use of the "raw" slope and the slope after applying an averaging and/or filtering algorithm, since both options are possible.

The slope of the measurement curve may be calculated in any way known to the skilled person. Thus, the slope may be calculated and/or derived by forming the first derivative of the measurement curve and/or by dividing a decrease and/or increase in the pressure values by the time period required for achieving this decrease or increase, respectively. Generally, for the measurement curve and/or the slope of the measurement curve, the full curves may be used or any curves derived therefrom. Thus, the measurement curve may comprise the raw values of the pressure values and/or may comprise an arbitrary curve generated by filtering and/or averaging the measurement curve, as will be outlined in further detail below. Thus, the pressure values may be acquired at a specific measurement frequency, wherein average values may be formed over a number of pressure values, such as over ten measurement values.

As used herein, the term automatically desirably refers to the fact that the evaluation unit itself is adapted to perform a specific action or function by itself, without the need of a user interaction. Thus, again, a software routine may be implemented in a processor of the evaluation unit which automatically compares the slope of the measurement curve to at least one endpoint threshold value. The endpoint threshold value may be stored in a data storage of the evaluation unit. Additionally or alternatively, the at least one endpoint threshold value may be modified by the user, such as by inserting the endpoint threshold value manually or via at least one electronic interface and/or via at least one human-machine-interface. Alternatively, the evaluation unit may determine the endpoint threshold value by itself. For instance, the endpoint threshold value may be derived on the basis of determined the noise level during the current or earlier measurements or it could be a particular fraction of the variation of the filtered or non-filtered measured pressure values during a particular period of measuring time. The term "compare" refers to the fact that an evaluation of one or more of the following conditions takes place: Is the slope of the measurement curve above the endpoint threshold value?; Is the slope of the measurement curve above or equal the endpoint threshold value?; Is the slope of the measurement curve equal to the endpoint threshold value?; Is the slope of the measurement curve below or equal the endpoint threshold value?; Is the slope of the measurement curve below the endpoint threshold value?. A specific type of condition may be predetermined. Therein, the slope of the measurement curve may fully be evaluated and compared to the at least one endpoint threshold value, and/or a specific part of the slope of the measurement curve may be compared to the at least one endpoint threshold value. Thus, typically, a first section of the slope of the measurement curve is disregarded when comparing the slope of the measurement curve to the endpoint threshold value, in order to disregard initial steep changes of the slope of the measurement curve. Thus, a time window of several milliseconds or even several seconds may be disregarded before starting the comparison of the slope of the measurement curve and the endpoint threshold value. Examples will be given in further detail below. Instead of comparing the slope of the measurement curve to the at least one endpoint threshold value, an absolute value of the slope of the measurement curve may be compared to the endpoint threshold value, in order to disregard a negative sign of the slope of the measurement curve when comparing the slope of the measurement curve to at least one endpoint threshold value.

As opposed to the standing pressure $p_{standing}$, which, in the art, is typically acquired at a predetermined point in time or at a predetermined time span after the position change of the user into the standing position and/or at a point in time arbitrarily determined by a therapist, the extended standing pressure allows for a precise and reproducible measurement. Thus, the comparison is performed such that the extended standing pressure $p_{standing, extended}$ is acquired at or after the endpoint, at which the slope of the measurement curve falls below a predetermined endpoint threshold value, which may indicate a significance of changes. Thus, the extended standing pressure may be measured at a point in time at which the measurement curve after the position change levels out or asymptotically approaches an endpoint value, which is more or less constant. Thus, the monitoring system, by determining the extended standing pressure $p_{standing, extended}$, may be capable of providing a significant increase in reliability and reproducibility of measurement, as opposed to conventional measurements. A user interaction and/or an interaction of medical staff, introducing a non-reproducible component of arbitrariness, may be avoided by automatically detecting the endpoint of changes in the measurement curve and, thus, using the pressure value acquired at or after the endpoint, indicating an endpoint of changes in the measurement curve, as the extended standing pressure.

The evaluation unit is adapted to automatically acquire the measurement curve of pressure values after the position change of the user. Thus, as an example, by monitoring pressure values over time, a start of the position change may automatically be detected, indicating that the above-mentioned measurement routine for determining the extended standing pressure will have to start.

Further, the evaluation unit may be adapted to acquire the resting pressure at least once before the position change. Thus, the resting pressure $p_{rest}$ may be acquired once or several times before the start of the above-mentioned measurement routine for determining the extended standing pressure. The resting pressure may be used as a baseline for subsequent measurements.

As outlined above, the position change is a position change of the user from a resting position into the standing position. The resting position may be a sitting position and/or a supine position. However, other types of position changes are possible.

As further outlined above, the endpoint is detected automatically, by subjecting the slope of the measurement curve to one or more conditions implying the at least one endpoint threshold value. Preferably, the at least one endpoint threshold value indicates an upper limit of tolerable changes of the measurement curve, below which the measurement curve is considered to be stable and/or is considered to have reached its asymptotic end value. Thus, the endpoint is automatically detected when the slope of the measurement curve is equal or below the endpoint threshold value.

The endpoint threshold value, specifically in the case this endpoint threshold value indicates a maximum tolerable change in the measurement curve, preferably may be a change in the measurement curve over time equal to or less than 1 mmHg per second, preferably equal to or less than 0.2 mmHg per second, more preferably equal to or less than 0.05 mmHg per second. However, other types of endpoint threshold values may be used alternatively and/or in addition.

The measurement curve and/or the slope of the measurement curve may be subject to at least one averaging and/or at least one filtering algorithm. The evaluation unit may be adapted to perform this averaging and/or filtering algorithm. Thus, preferably, the evaluation unit may be adapted to perform at least one of an averaging operation and at least one filtering operation on the measurement curve before comparing the slope of the measurement curve to the endpoint threshold value. As an example, an averaging operation may be used which generates a median over a predetermined number of pressure values, preferably over 3 to 20 pressure values, more preferably over 5 to 15 pressure values and most preferably over 10 pressure values. However, additionally or alternatively to generating a median, other types of averaging operations may be used, such as an averaging operation which generates a geometric mean and/or an arithmetic mean value.

As discussed above, the evaluation unit is adapted to determine at least one key figure K indicating the efficacy of the compression device. The key figure specifically may be determined by using pressure values provided by the pressure sensor. As used herein, the term key figure generally may refer to an arbitrary measure of efficacy of the compression system. Thus, the at least one key figure may directly or indirectly imply one or more types of information derived directly or indirectly from the pressure values, such as one or more pieces of information indicating the pressure exerted by the compression device onto the body part of the user. Additionally, the at least one key figure may directly or indirectly be indicative of one or more physiological parameters and/or body functions which are directly or indirectly linked to the compression therapy and/or the pressure exerted onto the body of the user by the compression device. Examples of key figures which may directly or indirectly be determined by using pressure values provided by the pressure sensor will be given in more details below.

Generally, the evaluation unit may be adapted to compare the key figure K to at least one efficacy threshold, such as a predetermined efficacy threshold and/or at least one efficacy threshold which may be provided by a user of the monitoring system, for automatically determining the efficacy of the compression device.

When using one or more key figures for determining the efficacy of the compression system, preferably, a plurality of different key figures may be used. Thus, specifically, the evaluation unit may be adapted to determine at least two different key figures $K_1$ and $K_2$. The evaluation unit may be adapted to automatically determine the efficacy of the compression device by a combination of the at least two key figures $K_1$ and $K_2$. Thus, the combination of the key figures $K_1$ and $K_2$ may generally comprise an arbitrary combination of these key figures and/or of one or more figures derived from these key figures $K_1$, $K_2$. Specifically, the evaluation unit may be adapted to perform at least one multivariate evaluation operation $f(K_1,K_2)$ using the key figures $K_1$ and $K_2$, wherein the evaluation operation is adapted to generate a statement on the efficacy of the compression device. As an example, a linear combination of $K_1$ and $K_2$ and, optionally, other key figures may be used.

The at least one key figure is selected from the group consisting of:

- the extended standing pressure $p_{standing,\,extended}$;

- an extended static stiffness index ESSI, the extended static stiffness index being determined by subtracting the resting pressure $p_{rest}$ from the extended standing pressure $p_{standing,\,extended}$;

- a difference $ESSI_1 - ESSI_2$ between at least two extended static stiffness indices $ESSI_1$ and $ESSI_2$, the extended static stiffness index $ESSI_1$ being determined by subtracting a first resting pressure $P_{rest1}$ from a first extended standing pressure $p_{standing,\,extended\,1}$, the extended static stiffness index $ESSI_2$ being determined by subtracting a second resting pressure $p_{rest2}$ from a second extended standing pressure $p_{standing,\,extended\,2}$;

- a ratio $ESSI_1 : ESSI_2$ of at least two extended static stiffness indices $ESSI_1$ and $ESSI_2$, the extended static stiffness index $ESSI_1$ being determined by subtracting a first resting pressure $p_{rest1}$ from a first extended standing pressure $p_{standing,\,extended\,1}$, the extended static stiffness index $ESSI_2$ being determined by subtracting a second resting pressure $p_{rest2}$ from a second extended standing pressure $P_{standing,\,extended\,2}$;

- a difference between at least two extended standing pressures $p_{standing,\,extended\,1}$ and $p_{standing,\,extended\,2}$ acquired at at least two different points in time;

- a ratio of at least two extended standing pressures $p_{standing,\,extended\,1}$ and $p_{standing,\,extended\,2}$ acquired at at least two different points in time;

The resting pressure, the standing pressure and the extended standing pressure have been discussed in detail above. The baseline resting pressure $p_{rest,\,baseline}$ generally is a resting pressure $p_{rest}$ measured directly after application of the compression system, such as within a time span of less than about 30 minutes after application of the compression system and allowing the compression system and/or the pressure sensor to settle, for example after the patient has freely moved around or has stood up at least once. While typically the resting pressure will be determined prior to standing pressure, it will be appreciated that it is possible to alternate the sequence where standing pressure is determined prior to resting pressure.

The extended static stiffness index ESSI is a new key figure which makes use of the extended standing pressure $p_{standing,\,extended}$. Thus, as compared to the conventional static stiffness index SSI, the extended static stiffness index is a more reliable key figure. Similarly, the difference between two different extended static stiffness indices $ESSI_1$ and $ESSI_2$ is more reliable than the conventional difference $SSI_1 - SSI_2$. Again, similarly, the ratio $ESSI_1 : ESSI_2$ is a more reliable and more reproducible key figure as compared to $SSI_1 : SSI_2$. However, the conventional key figures SSI, $SSI_1$ and $SSI_2$ may be used additionally or alternatively.

Further details regarding conventional measurements of the static stiffness index SSI are explained in the above-mentioned publication by H. Partsch et al.

As outlined above, each of the key figures and/or an arbitrary combination of the key figures may be compared to at least one efficacy threshold, such as for automatically determining the efficacy of the compression system. Exemplary embodiments of efficacy thresholds, which may be used within the present invention, will be given in further detail below.

The evaluation unit generally may be adapted to invite the user to perform at least one measurement routine for measuring the at least one key figure. Thus, the monitoring system may provide one or more optical and/or acoustical signals to the user to indicate that performing a specific measurement routine is advisable and/or indicating specific steps to be taken by the user in order to perform the measurement routine. Thus, as will be outlined in detail below, the evaluation unit may provide one or more display devices and/or acoustic output devices such as loudspeakers, allowing for an interaction with the user and allowing for indicating to the user the steps to be taken for performing the measurement routine.

The evaluation unit further may be adapted to generate at least one warning in case the key figure is detected to be outside an admissible range. Thus, one or more of the key figures or an arbitrary combination of the key figures may be compared to one or more thresholds indicating an admissible range for the respective key figures and/or combination of key figures. Thus, a warning may be generated in case a specific key figure is detected to be too high or too low. The at least one admissibility threshold may be predetermined and/or may be adaptable or determinable by the at least one user and/or by a medical staff. The at least one warning may be an acoustic and/or a visual and/or a haptic warning which may be output to the user, such as by visual indicia provided on a display device and/or a warning sound. Additionally or alternatively, an electronic warning may be generated, such as by providing an appropriate warning signal to another device, such as a patient monitoring system which is connected to the monitoring system. Thus, the monitoring system may be implemented into and/or may be part of a general medical system for patient care.

The monitoring system, as outlined above, preferably may comprise one or more user interfaces, allowing for providing information to the user and/or allowing for the user to input commands and/or information. Thus, the monitoring system may comprise at least one display element, and the evaluation unit preferably is adapted to provide instructions to the user via the display element. Thus, the evaluation unit may be adapted to provide instructions to the user which position to take. Additionally or alternatively, the display element may be adapted to output specific measurement information, such as one or more pressure values and/or one or more of the above mentioned key figures.

The monitoring system may further be adapted to lead the user through at least one measurement routine. Additionally or alternatively, the monitoring system may automatically detect that the user has taken a specific attitude and, correspondingly, may determine a specific key figure related to that attitude. Thus, by combining the attitude information with the pressure value, the evaluation unit may automatically determine that the user has taken a standing position and may determine the standing pressure and/or the extended standing pressure automatically, preferably without any further user interaction. Further, by combining the attitude information with the pressure value, the evaluation unit may detect changes in an attitude of the user, such as a position change from a resting position (preferably a supine position) into a standing position and may determine the standing pressure and/or the extended standing pressure automatically, preferably without any further user interaction. Further, the evaluation unit may detect a walking motion by evaluating the attitude information and may automatically determine at least one key figure related to a walking motion. Various further options are feasible.

As outlined above, the monitoring system, preferably the measuring device and/or the display and control device, may be adapted to provide acoustic and/or visual and/or haptic instructions to the user in order to indicate to the user which steps to take for performing the measurement routine. Thus, specific instructions regarding a position to be taken by the user may be provided. As an example, the user, in the measurement routine, may at least once take the resting position, wherein the resting pressure $p_{rest}$ is acquired at least once by the monitoring system. Further, in the measurement routine, the user at least once may take the standing position, wherein, in the standing position, the standing pressure $p_{standing}$ and/or the extended standing pressure $p_{standing,\,extended}$ are determined at least once.

As outlined above, the resting position preferably is a supine position. As used herein, the term supine refers to a dorsal position in which the user rests on a couch or lounger with his back, preferably with his knees flexed and his feet supported by the couch or lounger, respectively. Preferably, the legs are in a relaxed position.

As outlined above, the monitoring system, preferably the evaluation unit, may further be adapted to recognize at least one predetermined type of movement of the user by evaluating a measurement curve of pressure values. The measurement curve of pressure values may be the same as the measurement curve of pressure values used for the extended standing pressure $p_{standing,\,extended}$. Alternatively, a different measurement curve may be used. Thus, preferably, the evaluation unit may be adapted for determining a walking movement of the user by recognizing period changes of the pressure values in the measurement curve of pressure values. Further, the evaluation unit may be adapted to store an activity profile of the user. Thus, as used herein, the term activity profile of the user refers to an arbitrary amount of data indicating activity-induced pressure values or changes of pressure values, such as activity profiles determined or generated by walking movement and/or sports. The evaluation unit may further be adapted to use a pattern recognition algorithm for comparing a measurement curve of pressure values to a predetermined set of reference patterns. Again, the measurement curve of pressure values may be the same measurement curve of pressure values as used above for the purpose of determining the extended standing pressure $p_{standing,\,extended}$ and/or the measurement curve of pressure values as used above for recognizing at least one predetermined type of movement of the user. Additionally or alternatively, at least one separate measurement curve of pressure values may be used. As further used herein, the term reference pattern refers to a specific section of a measurement curve which may be stored in a data storage of the evaluation unit and which may indicate a specific type of activity of the user and/or which may indicate a specific physiological state of the user. Thus, by comparing the measurement curve to a predetermined set of reference patterns, a specific activity may be detected, such as a walking movement and/or any other type of predetermined activity. Additionally or alternatively, by comparing the measurement curve of pressure values to a predetermined set of reference patterns, one or more illnesses may be detected.

Further preferred embodiments may refer to the type of the at least one pressure sensor. As indicated above, the pressure sensor itself may comprise at least one sensing element and/or at least one sensing portion. The pressure sensor preferably may be selected from the group consisting of: a semiconductor pressure sensor; a pressure sensor having a deformation-sensitive resistor; a pressure sensor having a fluid-filled bladder. As an example, the at least one pressure sensor may comprise at least one fluid-filled bladder acting as a sensing portion, wherein the bladder may be located within the compression device or underneath the compression device, and at least one measurement portion located outside the compression device, such as in a pouch attached to the compression device, wherein the measurement portion and the sensing portion are fluidically connected via at least one tube. Thereby, a pressure may be transmitted from the sensing portion to the measurement portion. However, additionally or alternatively, other principles of measurement and/or other types of pressure sensors are applicable in addition or alternatively.

In a further preferred embodiment, the evaluation unit may be adapted to detect arterial pulsations in a measurement curve of pressure values provided by the pressure sensor. Again, the measurement curve of pressure values may be identical to one or more of the measurement curves disclosed above used for different purposes. Again, additionally or alternatively, a separate measurement curve may be used for detecting the arterial pulsations. For detecting the arterial pulsations, a frequency-based analysis of the measurement curve may be performed, such as a Fourier transformation. Additionally or alternatively, a filtering algorithm may be used, such as by filtering periodic pulsations in the measurement curve in a typical range of frequencies for arterial pulsations, such as in a range of 30 beats per minute to 200 beats per minute. The evaluation unit may further be adapted to generate a warning in case an amplitude of the arterial pulsations is below a predetermined safety threshold. Thus, again, an acoustic warning and/or a visual warning and/or a haptic warning and/or an electronic warning may be generated in case the amplitude of the arterial pulsations is below the predetermined safety threshold. The warning may indicate to the user or to the medical staff that the person is in a critical condition and/or that the compression device exerts an overpressure onto the body part of the user.

As outlined above, the evaluation unit preferably may comprise at least one processor. The at least one processor generally may include an arbitrary type of data evaluation device, including a microprocessor and/or a volatile or non-volatile data storage. Further, one or more electronic interfaces and/or one or more user interfaces may be comprised.

In a further aspect not being part of the present invention, a compression system for use in compression therapy is disclosed. The compression system comprises at least one monitoring system according to one or more of the embodiments disclosed above or according to one or more of the embodiments disclosed in further detail below. The compression system further comprises at least one compression device for exerting pressure onto a body part of a user.

As outlined above, the compression device preferably comprises at least one of: a compression bandage, a compression sleeve, a compression garment. Additionally or alternatively, other types of compression devices may be used. Most preferably, the compression device comprises at least one textile material such as at least one cloth and/or fabric and/or fiber material. Most preferably, the compression device comprises at least one flexible or elastic material, preferably having a specific stiffness.

Further, the compression device preferably is a passive compression device. As used herein, the term passive compression device refers to a device which is capable of exerting the pressure onto the body part of the user due to its elastic or flexible properties, in conjunction with a predetermined elongation and/or expansion of the compression device prior to application or during application. Thus, the passive compression device preferably is a compression device which does not include any type of actuator, such as a hydraulic or electric actuator.

As outlined above, the at least one body part generally may be or may comprise an arbitrary part of the body of the user. Thus, the body part preferably may be selected from the group consisting of: a leg of the user or a part of a leg of the user; a calf of the user; a thigh of the user; an arm of the user or a part of an arm of the user such as a forearm or an upper arm of the user; a finger or a finger digit of the user; a toe of the user; a foot of the user or a part of a foot of the user. Additionally or alternatively, other anatomical areas may be used for the compression therapy, in which compression and pressure measurements may be appropriate. The compression device may act on a single body part of the user or onto a plurality of body parts, such as on a thigh and on a calf and/or on both thighs and/or on both calves of the user.

In a further aspect not being part of the present invention, a method for determining the efficacy of at least one compression device for use in compression therapy is disclosed. The method preferably may make use of the monitoring system and/or the compression system as disclosed in one or more of the embodiments listed above or listed in further detail below. Thus, for specific embodiments of the method, reference may be made to the monitoring system and/or the compression system. However, other types of devices may be used.

In the method, at least one pressure sensor is used for measuring a pressure exerted onto a body part of a user by the compression device. Further, at least one attitude sensor is used for acquiring at least one attitude information of the user. The attitude information comprises an information on at least one of a position, an orientation and a movement of the user. At least one measuring device having at least one evaluation unit is used. The measuring device communicates with the pressure sensor and receives at least one pressure value acquired by the pressure sensor and at least one attitude information acquired by the attitude sensor. The evaluation unit automatically combines the pressure value and the attitude information in order to determine at least one key figure K indicating the efficacy of the compression device taking into account the attitude information.

The method preferably may be performed such that at least one resting pressure $p_{rest}$ with the user being in a resting position is acquired. Additionally or alternatively, at least one extended standing pressure $p_{standing,\ extended}$ with the user being in a standing position may be determined, by using the following procedure:

- a measurement curve of pressure values after a position change of the user into the standing position is acquired;
- a slope of the measurement curve is automatically compared to at least one endpoint threshold value and, depending on a result of the comparison, an endpoint of a change in the measurement curve induced by the position change is automatically detected, and a pressure value acquired at or after the endpoint is assigned to the extended standing pressure $p_{standing, extended}$.

The method preferably may imply at least one action on the basis of the resulting key figure K. As an example, the method may be performed such that the compression device is exchanged in case the compression device's efficacy is found to be below a predetermined threshold. As used herein, the term "exchange" generally may refer to a partial or complete removal of the compression device and a replacement of the compression device by a new compression device. Additionally or alternatively, the compression device may simply be fully or partially be re-adjusted, such as by unwinding a compression bandage, followed by a new application of the compression bandage.

For further details and optional embodiments of the method, reference may be made to the disclosure of the monitoring system and/or the compression system above or below. Thus, again, the method implies the determination of one or more key figures, as outlined above. Again, one or more of the key figures may be compared to one or more threshold values, such as to one or more safety threshold values. Again, a warning may be created in case one or more of the key figures may be too high or too low or out of range, such as in case the pressure exerted onto the body part is too high. Thus, appropriate warnings for excessive pressures such as baseline pressures, resting pressures, standing pressures, extended standing pressures and so on may be generated.

As an example, a normal range for the resting pressure $p_{rest}$ may be 10 to 70 mmHg, preferably 20 to 50 mmHg and, most preferably 25 to 35 mmHg.. In case the resting pressure is outside the named range, a warning may be created.

Additionally or alternatively, the standing pressure $p_{sianding}$ and/or the extended standing pressure $P_{standing, extended,}$ may be compared to one or more limit values. Thus, an admissible range for these standing pressures or extended standing pressures may be 30 to 120 mmHg, more preferably 40 to 100 mmHg and, most preferably, 45 mmHg to 65 mmHg.

Typically the time period needed to make an ESSI measurement depends on the particular patient's condition. For example for a patient having a severe venous insufficiency a measurement of ESSI may be completed in a short period e.g. as low as 30 seconds or even less, whereas a ESSI measurement for a healthy patient requires a longer period, e.g. up to 3 minutes

As outlined above, the method preferably may use the monitoring system according to one or more of the embodiments disclosed above and/or according to one or more of the embodiments disclosed in further detail below. Thus, in case the evaluation unit is disclosed to be adapted to perform a specific action, the method may imply an appropriate method step. Similarly, the monitoring system and/or the compression system may be adapted to perform a method according to the present invention.

As outlined above, the method preferably may be performed such that at least one key figure K is determined by using pressure values provided by the pressure sensor. The at least one key figure preferably may be a measure of efficacy of the compression device. As outlined above, the key figure preferably may be compared to one or more threshold values, such as to one or more predetermined threshold values. Most preferably, the compression device may be exchanged in case the compression device's efficacy is found to be below a predetermined threshold, such as in case a predetermined key figure is found to be out of range.

Short description of the figures

Further details of the invention may be derived from the following disclosure of preferred embodiments.

The features of the embodiments may be realized in an isolated way or in any combination. The invention is defined by the appended claims.

. The embodiments are schematically depicted in the figures. Identical reference numbers in the figures refer to identical elements or functionally identical elements or elements corresponding to each other with regard to their functions.

In the figures:

Figure 1A    shows an exemplary embodiment of a monitoring system and/or a compression system;
Figure 1B    shows a schematic cross-sectional view of a certain portions of the monitoring system and the compression system depicted in Figure 1A;
Figure 1C    shows a block diagram of exemplary set up on an exemplary measuring device
Figure 2    shows a user in a resting position;

Figure 3      shows a measurement curve of pressure values acquired after a position change into a standing position;

Figure 4      shows a slope of the measurement curve of Figure 3;

Figure 5      shows a measurement curve of pressure values acquired during an activity of a user;

Figure 6      shows a measurement curve including arterial pulsations of pressure values;

Figure 7      shows measurement curves including refilling curves of normal limbs and limbs with incompetent venous valves;

Figure 8      shows different positions where a pressure sensor may be positioned on the lower leg of a human;

Figure 9      shows a flow chart of an embodiment of a method for determining the efficacy of a compression device ;

Figure 10     shows a flow chart of an embodiment of a method ; and

Figure 11     shows a further exemplary embodiment of a monitoring system and/or a compression system.

Detailed description

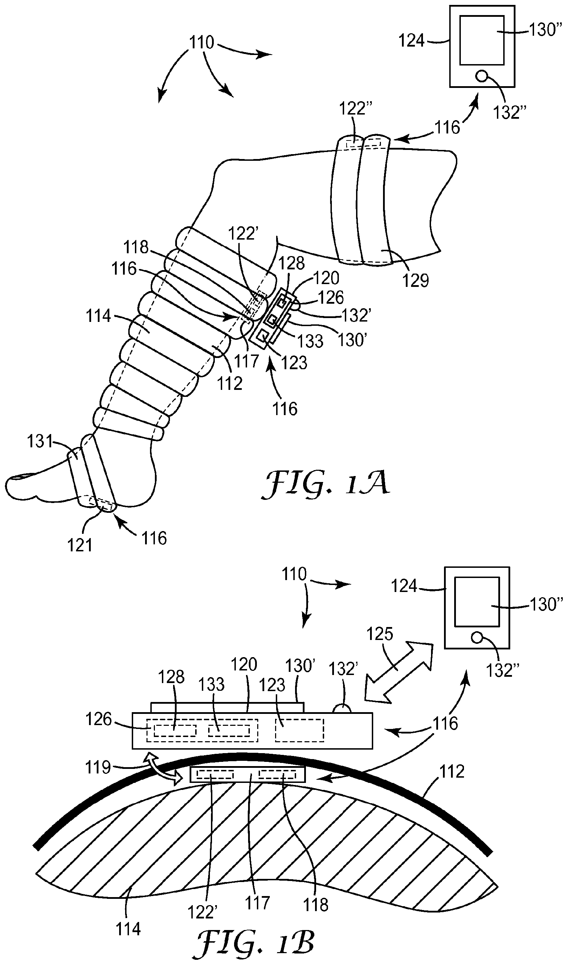

In Figure 1A, an exemplary embodiment of a monitoring system 116 for determining the efficacy of a compression device used for exerting pressure onto a body part of a user in the framework of compression therapy as well as an exemplary compression system 110 for use in compression therapy is depicted. The compression system 110 comprises at least one monitoring system 116 and at least one compression device 112 for exerting pressure onto a body part 114 of a user, such as a calf. The compression device 112, as depicted in Figure 1A, may preferably comprise a compression bandage. However, other types of compression devices may be used additionally or alternatively.

Figure 1B shows a schematic cross-sectional view of certain parts of the exemplary monitoring system 116 and compression system 112 shown in Figure 1A.

As shown in Figures 1A and 1B, the monitoring system 116 typically comprises at least one pressure sensor 118 for measuring pressure exerted by the compression device onto the body part of the compression device user, which may be placed in between the compression device 112 and the body part 114 of the compression device user (as shown in Figures 1A and 1B) and/or may be placed fully or partially inside the compression device 112, such as between two or more layers of the compression bandage. Such as pressure sensor may be part of a unit 117 that is configured and arranged to be placed between the compression device and the relevant body part of the device user or fully or partially within the compression device (e.g. between two or more layers of a compression bandage). For ease in description such a unit will be termed in the following a "sub-bandage unit".

As shown in Figures 1A and 1B, the monitoring system 116 further comprises at least one attitude sensor 122 for acquiring at least attitude information on at least one of a position, an orientation and a movement of the user.

Attitude sensors may orientation and/or acceleration sensors. Orientation sensors may be for example inclinometer or tilt sensors. Acceleration sensors may be gyroscopes.

As shown in Figure 1A desirably there is at least one attitude sensor associated with the body part on which the compression bandage is placed. In the illustrated exemplary embodiment, this sensor is located at/on the calf of the user and labeled 122' in Figures 1A and 1B. As depicted in Figures 1A and 1B, such an attitude sensor may be included together with a pressure sensor in a sub-bandage unit, so that a common sensing unit is provided. Alternatively, such an attitude sensor may be provided externally, e.g. on the outside the compression device, as a separate sensor or as part of a separate unit/device. In regard to the latter such a sensor may be incorporated into an external measuring device. In the exemplary shown in Figures 1A and 1B, there is provided a single attitude sensor, e.g. an orientation sensor, in association with the body part on which the compression bandage is placed. In order to obtain a higher level of attitude information, it may be favorable to provide two attitude sensors (e.g. an orientation sensor as well as an acceleration sensor) or even more attitude sensors in association with the body part on which the compression bandage is placed.

In order to obtain a yet higher level of attitude information in order to better determine a particular position, orientation and/or movement of the user at a given point time, the monitoring system, if desired may comprise one or more attitude sensors associated with a body part or body parts on which there is no compression bandage. For example, as shown in Figure 1A, the monitoring system may comprise beside an attitude senor at the calf (122'), an additional attitude sensor (e.g. an orientation sensor) to be located on the upper leg, e.g. on the thigh (122") of the user. Although not denoted in the embodiment shown in Figure 1A, the monitoring system may also comprise an attitude sensor on the foot of the user.

In addition to the at least one pressure sensor 118 and in addition to the at least one attitude sensor (122', 122"), the monitoring system 116 may, if desired, further comprise one or more additional sensor elements, such as at least one of a temperature sensor, a force sensor and an additional pressure sensor. Regarding the latter, a monitoring system may comprise one or more pressures for measuring a pressure exerted onto the body part which is not caused by the at least one compression device. Unlike the at least one pressure sensor that is used for measuring a pressure exerted onto a body part of a user by the compression device, such pressure sensors would be used for measuring a pressure exerted onto a body part of user due to other factors. For ease in distinction between these types of pressure sensors, such pressure sensors will be referred to in the following as second type pressure sensors. For example, in the exemplary embodiment in Figures 1A and 1B, such a pressure sensor 123 may be provided for measuring a pressure exerted onto the body part 114 from the outer side of the compression bandage, but which is not caused by the at least one compression device 112. Thus, an external or ambient pressure may be exhibited by the user's own weight when resting on a support. Such as a second type pressure sensor may be favorably used to determine "ambient" pressure in regard to pressure data collected by the pressure sensor 118 used to measure pressure exerted by the compression device. Accordingly, such a second type pressure sensor for measuring ambient pressure would be favorably located near or essentially at the same position as the pressure sensor underneath or within the compression bandage, with the exception that it is located "outside" of the compression bandage, such that the sensor does not measure pressure exerted by the compression bandage onto the body part. Such a pressure sensor may be appropriately attached to and/or integrated into the compression device, e.g. attached to or integrated onto an outer surface of the compression bandage. Such as pressure sensor may be incorporated into the measuring device described in more detail below. In order to obtain a different type of higher level information in order to better determine a particular position, orientation and/or movement of the user at a given point time, the monitoring system, if desired, may comprise one or more force sensors or one or more pressure sensors in association with a body part or body parts on which there is no compression bandage. For example, as shown in Figure 1A, the monitoring system may comprise either a force sensor or a second type pressure sensor 121 on the sole of the foot of the user. Such a sensor would allow for the measurement of force or pressure onto the sole of the foot, thus facilitating movement and/or position determination.

For sensors located separated from the at least one compression device, desirably there is provided at least one mounting element for mounting such sensors to a body or body part of the user. As shown in Figure 1A, the mounting element may be a short winding of cohesive wrap or adhesive tape (129, 131). Alternatively, sensors or more appropriately units comprising such sensors may be provided with an adhesive, e.g. skin-adherent adhesive, on one surface.

As shown in Figures 1A and 1B, the monitoring system 116 typically further comprises at least one measuring device 120 having at least one evaluation unit 126. The measuring device 120 is adapted to communicate with the at least one pressure sensor (118) and the at least one attitude sensor (122', 122") wherein the at least one evaluation unit is adapted to receive at least one pressure value acquired by the at least one pressure sensor and to receive at least one attitude information acquired by the at least one attitude sensor. In the event, a monitor systems comprises one or more other sensors, e.g. a force sensor or second type pressure sensor, desirably the measuring device is adapted to communicate with such sensor(s) wherein the at least one evaluation unit is adapted to receive at least one sensed value acquired by such sensor(s). The measuring device 120, in particular the evaluation unit 126, may comprise at least one processor 128, such as one or more microprocessors. Additionally, the measuring device, in particular the evaluation unit 126, may comprise one or more data storage devices 133, such as one or more volatile and/or non-volatile data storage devices.

Depending on the particular configuration of the system, communication between sensors and the measuring device, in particular the evaluation unit thereof may be achieved by hard (e.g. wire) connection or wireless. In the exemplary embodiment shown in the Figures 1A and 1B, communication between the sensors and the measuring device, in particular the evaluation unit, for the most part is wireless. For example, communication between the measuring device 120 and pressure and attitude sensors (118,122') located at the calf under the compression device is favorably wireless, preferably via RFID, more preferably according to ISO/IEC standard 15693-3. For this reason, as shown in the exemplary embodiment depicted in Figures 1A and B, the measuring device is typically located near or essentially at the same position as the sensor(s) underneath or within the compression bandage, with the exception that the measuring device is located "outside" of the compression bandage. Accordingly the measuring device may be appropriately attached to and/or integrated into the compression device, e.g. attached to or integrated onto an outer surface of the compression bandage. Besides RFID type communication it will be understood other ways of wireless communication are possible, such as Bluetooth and/or radio transmission. In regard to the exemplary, additional sensors positioned at the upper leg and at the foot, again communication between the measuring device and said sensors is favorably wireless, preferably via Bluetooth Smart. It will be appreciated that such sensors will be typically provided in the form of sensor units, each including besides the sensor(s) a power supply (e.g. battery) as well as the appropriate elements (e.g. antenna, etc.) for the selected form of wireless communication. In regard to the exemplary second type pressure sensor 123 located at the calf outside the compression device and incorporated into the measuring device 120, communication is typically achieved via hard connection(s). Similarly for aforesaid mentioned alternative embodiments where the least one attitude sensor associated with the body part on which the compression bandage is placed (e.g. the attitude sensor 122' positioned at the calf) is positioned outside the compression device (rather than underneath or within the compression device) and incorporated into the measuring device, communication may then be achieved via hard connection(s).

The monitoring system may comprise at least one display element (such as one or more segmented displays and/or one or more matrix displays) and/or at least one interface (e.g. user interfaces (such as at least one keypad or push button and/or other types of user interfaces e.g. allowing for a user to input commands into the evaluation unit), electronic interfaces (such as interfaces for other devices, e.g. charging outlet, USB) and/or data interfaces (interfaces for transferring data in and out of the system).

For example, the measuring device, in particular the evaluation unit thereof, may further comprise at least one display element and/or at least one interface, e.g. a user interface, a electronic interface and/or a data interface. In this regard, as shown in the exemplary embodiment illustrated in Figures 1A and B, the measuring device 120 desirably includes a display element 130' and an user interface 132'.

Alternatively or in addition, the monitoring system 116 may further comprise at least one display and control device, e.g. as a separate unit from the measuring device. Such a device would desirably comprise at least one display element and/or at least one interface, e.g. a user interface, a electronic interface and/or a data interface. For example, the exemplary embodiment illustrated in Figures 1A and B comprises a display and control device 124 which includes a display element 130" and at least one user interface 132". Such a display and control device 124 is preferably adapted to wirelessly communicate with the measuring device 120. In Figure 1B, the wireless communication symbolically is denoted by reference number 125. Preferably, the wireless communication may comprise at least one Bluetooth communication. Additionally or alternatively, other types of communication are feasible, both wire-bound and wireless. The display and control device 124 preferably may be a hand-held device. As an example, the display and control device 124 preferably may be or may comprise a mobile communication device such as a smart phone. Thus, the display and control device 124 preferably may comprise at least one display and/or at least one user interface. Further, the display and control device 124 preferably may comprise processing capabilities, such as at least one processor.

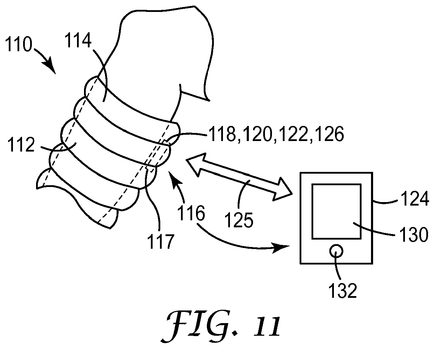

As already indicated above, the compression system 110 and/or the monitoring system 116, in particular the individual components thereof, may be embodied in various ways. Figures 1A and B show a single exemplary embodiment of such a system. A number of alternative configurations may be envisioned. For example, as already mentioned above the attitude sensor associated with the body part on which the compression device is placed (e.g. the attitude sensor positioned at the calf (122')) may be provided externally, e.g. on the outside the compression device, as a separate sensor or as part of a separate unit/device, e.g. an external measuring device. Alternatively, the measuring device could be placed underneath or within the compression device (e.g. between the compression device and the body part of the compression device user may be placed fully or partially inside the compression device, such as between two or more layers of the compression bandage). For example, such an embodiment is shown in Figure 11. Moreover, in the exemplary embodiment shown in Figure 11, the measuring device 120 with its evaluation unit 126 is provided together with the pressure sensor 118 and the attitude sensor 122' in a single sub-bandage unit 117. While the measuring device of such an embodiment could comprise at least one display element and/or at least one user interface, generally it is preferred that such an embodiment would include an external display and control device 124 including at least one display element 130 and/or at least one interface, e.g a user interface 132, said display and control device being in communication 125 (e.g. wireless communication) with the measuring device 120, in particular the evaluation unit 126. Such a display and control device can be like that described above and below. Although not depicted in Figure 11, such an exemplary embodiment could include one or more additional sensors, for example attitude, force, second type pressure sensors positioned appropriately at the thigh and/or foot similar to that shown in the embodiment of Figures 1A and B as well as an second type pressure sensor positioned outside the compression device at the calf for measuring ambient pressure as discussed above, where such sensors would be in communication (e.g. by hard connection or wireless) with the measuring device, in particular with the evaluation unit.

As outlined above, monitoring systems and compression systems and methods described herein may be adapted to gain metrics for determining the efficacy of the compression device, such as compression bandage efficacy. Metrics of efficacy may be a static stiffness index SSI and/or an extended or modified static stiffness index and/or any other key figure as disclosed above or as disclosed in further detail below. The evaluation unit 126 of the monitoring system 116 is adapted to automatically combine at least one pressure value measured by the pressure sensor 118 and at least one attitude information acquired by the attitude sensor 122 in order to determine at least one key figure K indicating the efficacy of the compression device 112, by taking into account the attitude information. Thereby, as an example, the key figure may be determined out of every day movements of the patient without the need of measurement procedure.

The monitoring system 116 may form an intelligent system to determine the conditions for measurements to extend battery life time. The at least measuring device 120 may communicate with the at least one pressure sensor 118 and, optionally, with one or more further sensors such as the at least one attitude sensor 122. The communication preferably takes place wirelessly, preferably by using RFID-technology, such as according to the ISO 15693 standard. In case a plurality of sensors is used, such as a plurality of pressure sensors 118, the communication of the measuring device 120 with the plurality of sensors may use several ways of communication, such as a wireless communication with at least one first pressure sensor 118 and a wire-based communication with at least one second pressure sensor 118. In this embodiment or in any other embodiments, the at least one pressure sensor 118 and one or more further sensors, such as the at least one attitude sensor 122, may be comprised in one or more sensor tags. The monitoring system 116 may furthermore power one or more of the sensors, such as the at least one pressure sensor 118 and/or the at least one attitude sensor 122, preferably wirelessly, such as by using the RFID field. The measuring device 120 may convert the measured values into pressure values, may compute the efficacy of the compression device 112 and may optionally determine safety features and may optionally alarm the patient. The sensor tag may be controlled by the measuring device 120 and may measure the pressure below the compression device 112, such as below the compression bandage.

The display and control device 124 might be a standard device like a personal computer, a laptop, a smart phone, a table PC or a similar device. It may further be a custom device optimized to the needs of the user. The display and control device 124 may form a user interface, by which the user may check the status of the compression device 112, may check the status of the compression system 110 and may check the measurement results. For example, the user may configure the monitoring system 116 and may start and stop measurement procedures. The display and control device 124 may be connected to the measuring device 120 preferably by using wireless standards like Bluetooth or Bluetooth low energy. Further, optionally, the display and control device 124 may be connected using a wire-based connection, such as a USB connection. Using the display and control device 124, a connection to a database to store measurement results or compression device data may be possible, too. The measuring device 120 may be used without the display and control device 124 as well.

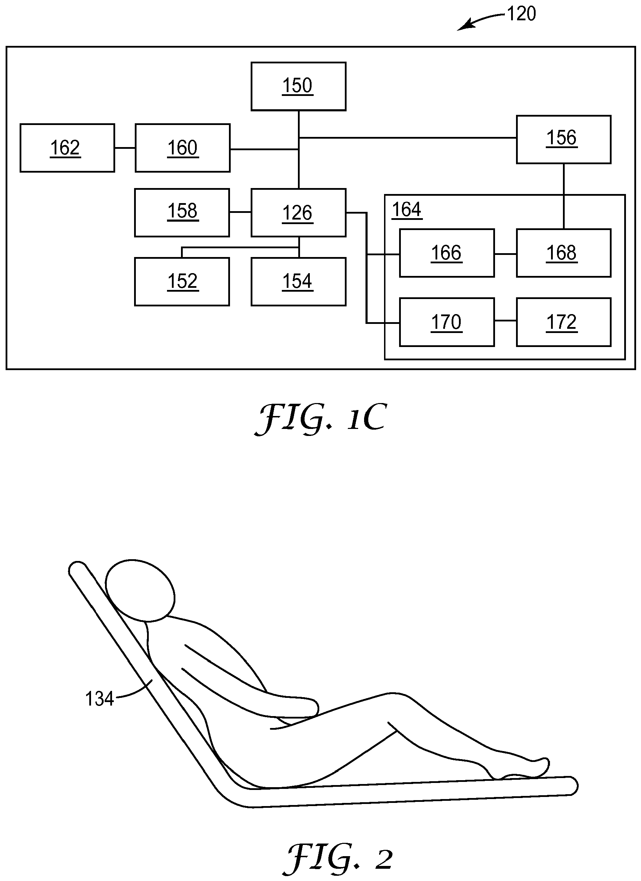

In Figure 1C, a detailed, potential, exemplary setup of a measuring device 120 is depicted. As disclosed therein, the measuring device 120, besides the evaluation unit 126 (which preferably comprises one or more processors and/or one or more data storage devices) or as a part of the evaluation unit 126, the measuring device 120 may comprise a memory 150. Further, one or more human-machine interfaces and/or machine-machine-interfaces may be provided, wherein, for the latter, both wireless and wire-based options are feasible. In Figure 1C, an input 154 and an output 156 are provided. The measuring device 120 may further comprise one or more power supplies 156, such as one or more electric energy storage device like batteries, accumulators or the like. Additionally or alternatively, a wire-based power supply may be provided, such as at least one an electric plug.

The measuring device 120 may further comprise one or more sensors 158 of its own, such as one or more temperature sensors or the like.

Further, the measuring device 120 may comprise one or more communication modules for communicating with the at least one pressure sensor 118 and, optionally, with further sensors such as the at least one attitude sensor 122, and, optionally, for communicating with the display and control device 124. In the exemplary embodiment of figure 1C, an RFID-IC 160 and an RFID antenna 162 are provided. For communicating with the display and control device 124, at least one communication module 164 may be provided, which may comprise one or more of a USB module 166 with a USB connector 168 and a Bluetooth module 170 with a Bluetooth antenna 172. Other embodiments are feasible.

The measuring device 120 preferably may further comprise at least one real time clock, preferably as a part of the evaluation unit 126. The real time clock may be used to determine the time of the day, the time period since the last measured value has been taken etc., for power saving purposes and to determine the status of the patient.

As outlined above, the monitoring system 116 comprises at least one pressure sensor 118 and at least one attitude sensor 122. These sensors as well as further, optional sensors may be integrated fully or partially into the measuring device 120, as schematically depicted in Figure 1C or may fully or partially be located outside the monitoring device. Thus, a motion sensor and/or a gyro-sensor (gyroscope) may be provided. The sensors may generally be used for detecting the status and/or condition of the patient, the status of the sensor and to enable efficient power saving techniques.

As outlined above, one or more power supplies 156 may be provided. The measuring device 120 may be powered by an integrated accumulator or, when connected to a USB host or a USB charging device, measuring device 120 may be powered by the USB connection. This USB connection can be used to charge the accumulator, too.

The measuring device 120 may comprise one or more memories 150, such as one or more volatile or non-volatile data storage devices. Preferably, a non-volatile memory, such as a flash memory, may be used to store measurement results.

Regarding the input 152, one or more inputs like pushbuttons may be provided. Additionally, input options may be given via the display and control device 124. The input facilities may be used for one or more of the following purposes: to e.g.

- turn the measuring device 120 on and off,
- initiate a connection to the display and control device 124, preferably a wireless connection,
- indicate certain patient's attitudes, such as patient's positions,
- indicate the necessity for replacement of the compression device 112.

Regarding the output 154, in addition to the optional output options provided by the display and control device 124, the measuring device 120 may provide one or more outputs like e.g. LEDs, one or more small display (e.g. LCD), and/or one or more acoustic outputs. The output 154 may be used to show one or more of:

- a device status of the measuring device 120 such as

  o on/off,
  o battery status,
  o sensor tag connected?
  o measurement in progress,

- a status of the compression device 112,
- warnings.

These outputs may be used to guide a patient through a measurement process.

The communication module 164 or communication block may comprise the USB module 166 or USB block, which may be used to establish a wire-based connection to the optional display and control device 124, to power the measuring device 120 and to charge the accumulator. Further, one or more wireless communication modules or wireless communication blocks may be provided, such as the Bluetooth module 170, preferably a Bluetooth LE block.

The measuring device 120 may have the following functions:

1) Permanent pressure measurement:
The measuring module 120 may read the pressure from the sensor tag via RFID.
2) Algorithms to determine the at least one key figure for determining the efficacy of the compression device 112, such as out of every day movements

Examples for determining key figures will be given in further detail below. Thus, an algorithm may be used which detects at least one standing pressure and at least one resting pressure. The detection of the resting pressure may be kept quite simple, such as by using a minimum pressure value detected as the resting pressure. To detect the standing pressure, the maximum pressure achieved with an asymptotic behavior may be assumed to be the standing pressure. This value may be smaller or equal to the standing pressure acquired from measurement process as you cannot be sure that the patient has been in a position to measure the standing pressure. As the standing pressure is the highest achievable pressure with an asymptotic behavior, the value derived from the everyday pressure measurements must be smaller. These two methods may be applied to the pressure values of a defined time interval (e.g. last 6h, 12h, 24h) at defined points of time. With the status/condition, which is detected with other sensors, this measurement can be further improved.

Thereby, as will be outlined in further detail below, a modified or extended static stiffness index ESSI may be determined as one example of a key figure indicating the efficacy of the compression device 112. If the ESSI has an insufficient quality, the patient might perform a test procedure. This procedure might be optimized and/or modified, such as by skipping some parts depending on the figures derived from the everyday measurements.

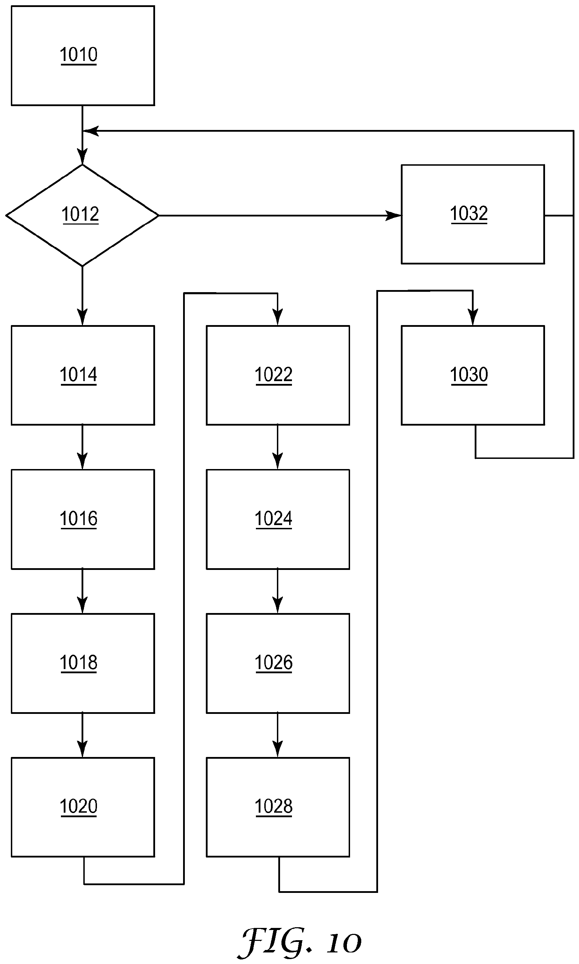

In Figure 10, a flow chart of an exemplary embodiment of operation of the monitoring system 116 is depicted. Therein, the following steps may be used:

1010    start
1012    reference measurement
1014    get sensor values
1016    preprocessing (e.g. compute pressure)
1018    determine patient attitude, e.g. patient position
1020    save sensor values and intermediate results
1022    calculate key figure K of compression device efficiency (e.g. ESSI)
1024    store key figure K
1026    display key figure or other results and/or alarm
1028    calculate sleep interval
1030    sleep mode
1032    guided measurement

In the following, some of the steps of Figure 10 are outlined in further detail:

Get sensor values:

Sensor values may be read from existing sensors:

- Pressure sensor below compression bandage
- Pressure sensor between reading device and compression bandage
- Acceleration sensors located at

    o Pressure sensor
    o Thigh
    o ...

- Tilt Sensor located at

    o Pressure sensor
    o Thigh
    o ...

- Temperature sensor

Preprocessing of sensor data:

- Improve quality of sensor data e.g. using median, average, kalman filter, ...
- Calculate Pressure below compression bandage

    o Use sensor value of pressure measurement below bandage
    o Error compensation using pressure measured between compression bandage and reading device

- Calculate features like

    o movement of the leg in polar coordinates,
    o gradient of movement (accelerating or slowing down)

Determine patient's attitude such as patient's position:

As outlined above, the key figure is determined by taking into account an attitude of the patient, such as a current attitude. As an example, one or more activities and/or body positions may be detected automatically by the monitoring system 116, such as:

- a supine position
- a standing position
- a walking movement
- a specific movement other than a walking movement.

As will be outlined in further detail below, the supine position, as depicted in Figure 2, may be used to measure a sub-bandage pressure without relevant venous filling and low muscle tone. Under controlled situations the patient might be instructed to be in a position as described in Figure 2, resting on a bed 134 or mattress. The knee is in slight flexion and the lower leg has no direct contact to the mattress. An automatic system might detect this position by a gyro-, motion- and, optionally, the pressure sensor:

- the gyro-sensor indicates a horizontal position
- the motion sensor indicates a low acceleration or no acceleration
- the pressure sensor indicates a low pressure, wherein a patient-specific evaluation regarding pressure levels may be performed

Therein, the sensor area should not have contact to the bed 134, mattress or armchair since, in this case, an increased pressure might occur. Further, an ankle joint position might be relevant for sub-bandage pressure, since, typically, 90% flexion increases pressure compared to relaxed plantar flexion.

In a standing position, the gyro-sensor typically detects a vertical position. The motion sensor typically detects no or minimal acceleration. The pressure sensor typically records high pressure. Therein, the following issues might be considered for evaluation:

- The weight bearing leg often shows higher pressure
- The time to complete venous filling without any movement often is crucial
- The sensor typically will not differentiate between a sitting and standing position
- 90° or more knee flexion in sitting position can show higher pressure than standing
- A supine position with 90° flexion of hip joint and 120° in knee joint might give higher pressure levels, than in the standing position

A further attitude the patient may take is a walking motion or any other type of defined movement. Due to muscle pumping action of the foot, the calf, the thigh and the hip muscles as well as the related joint pumps, the venous blood column will typically be proximally shifted, and the ambulatory venous pressure will typically be reduced. Also, joint movement, especially of the ankle joint, will typically transmit forces due to the typically low elasticity of the compression device 112 such as the compression bandage. The gyro sensor typically detects slightly changing but roughly vertical positions. The acceleration sensor typically detects regular accelerations, wherein the monitoring system 116 might undergo a learning process. The pressure sensor generally detects regular changes in chronological agreement to motion sensor. When determining a movement, it should be considered that a walking acceleration might typically show variations due to some factors, such as age of the patient, obesity, oedema, reduced joint mobility, foot wear and other factors, which might be taken into account when evaluating the patient's attitude.

Summarizing some of the findings above, Table 1 shows some examples of combining results from several sensors for determining the patient's attitude, such as the patient's current attitude. Therein, when referring to angular positions, such as vertical or horizontal positions, the term "substantially" may refer to the fact that slight deviations from the exact angular position might be tolerable, such as deviations by no more than 20°, preferably by no more than 10°.

*Table 1: Examples of sensor combinations for determining patient's current attitude.*

| Attitude | Position sensor (calf) | Position sensor (thigh) | Acceleration sensor (calf) | Pressure under bandage (calf) | force sensor under foot |
|---|---|---|---|---|---|
| **Sitting** | Substantially vertical | Substantially horizontal | Little | Medium | Low |
| **Lying** | Substantially horizontal | Substantially horizontal | Little | Low | Low |
| **Standing** | Substantially vertical | Substantially vertical | Little | High | High to Low |
| **Kneeling** | Substantially horizontal | Substantially horizontal OR substantially vertical | Little | Medium to very high | Low |
| **Walking** | Substantially vertical | Substantially vertical | Dynamic | Alternating | Alternating |

Further referring to the optional method steps depicted in Figure 10, the following options may be added:

Save sensor values and intermediate results:

The computed values and the measurement results may be stored with the current time stamp to the memory 150, such as to a non-volatile memory.

Calculate key figure K of compression device efficiency (e.g. ESSI):

Using the intermediate results and sensor data, at least one key figure K is determined, indicating the efficacy of the compression device 112. Some examples of key figures which may be determined, taking into account the patient's attitude, preferably the current attitude, will be outlined in detail below.

Store key figure K:

The key figure K may be stored in the memory 150, such as the non-volatile memory. When optionally connected to a database by using the display and control unit 124, the results may be stored in the database. This database might be accessible by medical staff, such as persons which might control the status of the patient and the compression device 112 online.

Display key figure K or other results and/or alarm:

The measured values and calculated results, such as the at least one key figure K, as well as further results, such as

- is the compression device 112 efficient or not,
- the current pressure,
- the patient's attitude such as the patient's position

or other results may be displayed via the display element 130 of the measuring device 120 and/or, optionally, when connected to the display and control device 124, via the display and control device 124. If one or more of the results are found to be out of a predetermined range, such as in case one or more key figures K are found to be above or below predefined thresholds (e.g. ESSI to low, pressure to high), the patient may be alarmed using a vibration alarm, acoustic feedback or using the display and control device 124. If necessary, other persons like doctors, nurses etc. can be alarmed as well, when the measuring device 120 is connected to the display and control device 124.

Calculate sleep interval:

To save energy and to extend the battery life, the measuring device 120 may not measure continuously but may switch into a sleep mode based on environmental conditions and measured values. The sleep interval might be extensive, such as lasting several hours, specifically if the last measurement of compression device efficacy (such as ESSI calculation) has been successful and has proven that the compression device 112 is still effective. If not, the sleep interval generally may dependent upon the current patient activity. If the patient is standing up, i.e. changing from a resting position to a standing position, a continuous measurement may be performed, until the pressure has settled. Then, the standing pressure may be detected, or the patient may perform other movements.

Sleep:

During the sleep phase the compression system 110 including the monitoring system 116 may sleep for a predefined sleep interval. This interval may be interrupted, if the patient is moving, such as due to an interrupt generated by an acceleration sensor and/or a tilt sensor. Then, a new measurement may be performed. The sleep interval may further be interrupted by the user, too, such as via an input at the measuring device 120 and/or at the display and control device 124.

Guided Measurement:

In the optional guided measurement, the patient may be guided, such as by one or more of:

- visual and/or optical commands from the measuring device 120
- visual and/or optical commands from the display and control device 124,

in order to perform a measuring sequence. When measuring one or more of the key figures, such as for measuring the ESSI as explained in detail below, the patient may have to take a resting position, and the resting pressure may be determined. When this is done, the patient may have to stand up, to measure the standing pressure. A guidance regarding the attitude to be taken by the patient may be provided by the measurement device 120 and/or by the display and control device 124.

The evaluation unit 126 preferably is adapted to perform a method for determining the efficacy of the compression device 112. As outlined above, the evaluation unit 126 is adapted to acquire at least one resting pressure $p_{rest}$ with the user being in a resting position. The evaluation unit 126 is further adapted to determine at least one extended standing pressure $p_{standing}$, extended with the user being in a standing position, by using the following procedure:

- the evaluation unit 112 acquires a measurement curve of pressure values after a position change of the user into the standing position;
- a slope of the measurement curve is automatically compared to at least one endpoint threshold value and, depending on a result of the comparison, an endpoint of a change in the measurement curve induced by the position change is automatically detected, and a pressure value acquired at or after the endpoint is assigned to the extended standing pressure $p_{standing, extended}$.

In the following, several embodiments of the method are disclosed which are suited to assess if the compression system 110 and, specifically, the compression device 112, are effective. Initial efficacy may be measured directly after application of the compression system 110 (baseline measurement).

In the following, pressures, key figures or time-related data determined during initial measurement will be indexed with a "1". The system may store baseline measurements of a patient together with user-specific information, such as a specific RFID ID-code assigned to the compression device 112 and/or the pressure sensor 118.

After hours or days it may be expected that compression properties of the compression device 112 change, potentially resulting in therapeutically inefficacy. Time, pressure data and key figures determined after some time of wearing will be indexed with a "2". Via RFID ID-code one or more consecutive values may be compared with baseline data.

According to current medical standard, typically, sub-bandage pressures and key figures are used as a surrogate marker for bandage efficacy, also called bandage efficacy. However, these pressure values are measured manually. That means the therapist decides at which exact time the resting or standing pressure is measured. However, values like the standing pressure show significant changes over time of assessment. Also, uneven pressure curves will not be smoothened, nor are there any automatic calculations of mean or average values to increase reproducibility. In contrary, algorithms allow appropriate detection e.g. for resting pressures or dynamic changes like pressure amplitudes.

Several methods to measure compression efficacy will be proposed in the following. Beside the measurement of efficacy of the compression device 112, such as the compression bandage, also other parameters like venous refilling time (section B) or safety of the bandage (section A and E) can be measured by the compression system 110 and, specifically, by the monitoring system 116.

Besides assessing of the compression system 110 and, specifically, the compression device 112, within predetermined time intervals, e.g. clinical visits or daily nursing service, efficacy can also be assessed continuously.

Several assessments can be done to judge efficacy or safety over the time of compression application. This continuous measurement can be done in the domestic environment or during normal activity of the user, also referred to as the patient. Further description is outlined under G-K.

A.) Measurement of the resting pressure $p_{rest}$

A first version of measuring the efficacy of the compression system 110, specifically of the compression device 112, is a measurement of the resting pressure $p_{rest}$. The resting pressure $p_{rest}$ describes the forces which are built up only by the compression system 110. The resting pressure specifically may be a supine pressure, i.e. a pressure taken with the patient in a supine position as depicted in Figure 2. The resting pressure specifically may be reduced over time due to material fatigue, slippage of bandage or limb volume reduction.

For measurement of the resting pressure $p_{rest}$, the patient has to be in a relaxed position, such as in a sitting or a lying position, also referred to as a supine position, as shown in Figure 2. Preferably, during the measurement of the resting pressure, the foot rests relaxed on a bed 134 or couch, wherein the knee of the patient preferably is slightly flexed and the calf preferably is completely free of the bed surface.

The measurement preferably may be activated by activating a pushbutton, keypad or touch screen, such as by using one or more of the user interfaces of the evaluation unit 126. In the following, pressure values are acquired by using the pressure sensor 118. The pressure values may be acquired over time intervals, such as time intervals of 1 second, with time interval, such as 10 measurements every 100 ms per time interval. The pressure values may be stored by the evaluation unit 126. As an example, averaged pressure values over the time intervals may be calculated and stored. Thus, an averaged value of the pressure values over the time interval may be calculated and stored, such as a geometric mean value, an arithmetic mean value or a median over the ten pressure values within each time interval of 1 s.

Optionally, the averaged pressure value, such as the median value, may be compared from one interval to the next time interval. If a pressure variation within 5 consecutive time intervals is below a specific threshold, such as below 1 to 10 %, preferably below 2 %, the resting pressure may be stored by the evaluation unit 126.

Thus, generally, in this embodiment or other embodiments of the present invention, the resting pressure $p_{rest}$ may be measured after a period of stabilization of a measurement curve of pressure values acquired using the pressure sensor 118, such as in case the variation of pressure values is below a predetermined threshold, such as a threshold of 1 to 10 %, preferably below 2 %. Therein, in this embodiment or other embodiments, the full measurement curve may be evaluated or an averaged measurement curve, such as a measurement curve containing pressure values averaged over a time interval and/or averaged over a number of measurement points.

To shorten the time required for the measurement and/or to avoid having to wait until a threshold value is reached, the median of the previous 5 time intervals can be calculated upon command (for example via push button) or automatically (for example if a certain, select measurement time (e.g. 2 minutes) has elapsed. The median is then stored as the sub-bandage resting pressure.

Generally, as outlined above, other time intervals may be used. Thus, in this embodiment or in other embodiments, instead of a time interval of 1 second, a shorter time interval, such as a time interval of 0.1 s, or a longer time interval, such as a time interval up to 60 s, may be defined.

Instead of 10 measurements within each time interval, also a different number of measurements within each time interval may be taken. Thus, a number of less than 10 measurements, such as 3 measurements, or a number of more than 10 measurements, such as up to 1000 single measurements, may be taken.

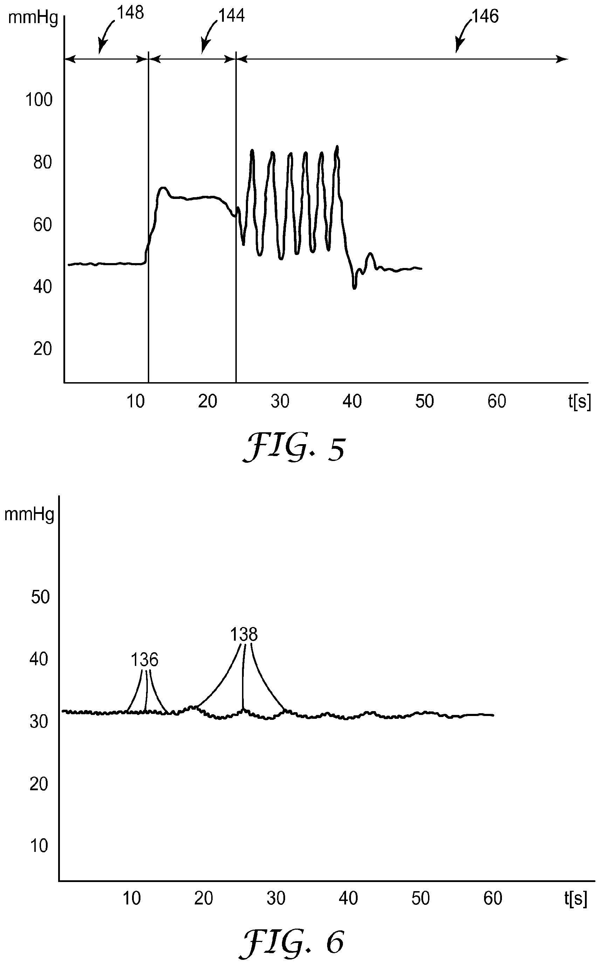

In Figure 6, a typical measurement curve of pressure values taken over a normal period of time, without any major position changes, is shown. Therein, on the vertical axis, the pressure values are given, provided in mmHg, wherein the horizontal axis is the time axis t.

As can be seen, the measurement curve of pressure values typically shows physiological periodic alterations due to the arterial pulsation (reference number 136) and due to the respiratory activity (reference number 138). As outlined above, it is possible to detect these arterial pulsations and to detect the amplitude of these pulsations. It is even possible to evaluate the amplitude of these pulsations and to compare this amplitude to a threshold, in order to be able to generate a warning in case the amplitude of the arterial pulsations is too low. Further, in view of the above-mentioned averaging over a plurality of pressure values, the arterial pulsations generally show that for analysis of arterial pulsation a minimum number of single measurements for averaging is desirable at least six per second. It will be appreciated that the higher the number, the more accurate the arterial pulsation analysis.

The above-mentioned measurements mainly refer to a measurement of the resting pressure $p_{rest}$, specifically an initial measurement of the resting pressure as a baseline measurement. Further, as outlined above, at least one standing pressure $p_{standing}$ is measured, specifically at least one so-called extended standing preSSUre $p_{standing, extended}$.

For measuring the standing pressure, after the measurement in the supine position, the system generally may invite the user to perform the measurement of the standing pressure, such as by inviting the user to change position into the standing position. For this purpose, the evaluation unit 126 may give an acoustical or numerical signal. This signal may be also used to remind changing the position to standing.

Directly after application of the compression device 112, the resting pressure $p_{rest1}$ may be too low or too high because of a false application technique. In order to detect this false application technique, the resting pressure $p_{rest1}$ may be compared to one or more threshold values. As an example, if the resting pressure $p_{rest1}$, preferably the supine resting pressure, is below 50 mmHg, the monitoring system 116, specifically the evaluation unit 126, may indicate that the compression is ineffective and has to be changed. This threshold can also be lower as detailed below:

$$P_{rest1} < 50 \text{ mmHg (preferably} < 20 \text{ mmHg, more preferably} < 15 \text{ mmHg, most preferably} < 10 \text{ mmHg)} \rightarrow$$
change compression, pressure too low

Also, as indicated above, $p_{rest1}$ may be too high. Typically, values higher than 60 mmHg are considered to be intolerable or may cause circulatory disorder:

$$P_{rest1} > 60 \text{ mmHg (more preferably} >80\text{mm Hg, most preferably} >100 \text{ mmHg)} \rightarrow \text{change compression,}$$
pressure too high

Generally, the resting pressure, such as the resting pressure measured in a supine position, decreases over time due to material fatigue, slippage of bandage or limb volume reduction. This process may also be monitored by comparing one or more key figures to one or more threshold values. Thus, in case some time after application of the compression device 112 the resting pressure $p_{rest2}$ drops below a threshold such as below 40 mmHg, the evaluation unit 126 may indicate that the compression device 112, such as the compression bandage, is not effective any longer. This threshold can also be lower as indicated below:

$$p_{rest2} < 40 \text{ mmHg (preferably} <15 \text{ mmHg, , more preferably} <25 \text{ mmHg, most preferably} < 5 \text{ mmHg)} \rightarrow$$
change compression

The resting pressure could also increase over time e.g. due to changes (e.g. slippage) in the compression

system such that $p_{rest2}$ could be too high (e.g. values higher than 60 mmHg):

$$P_{rest2} > 60 \text{ mmHg (preferably 80mmHg, most preferably 100mmHg)} \rightarrow \text{change compression, pressure too high}$$

As the resting pressure such as the supine pressure may show relevant inter-individual variations, a further option may be to calculate the absolute change or the relative change of the actual $p_{rest2}$ in comparison to the initial $p_{rest1}$. Thus, again, the absolute change of the relative change may be compared to one or more threshold values.

As an example, if $p_{rest2}$, in comparison with $p_{rest1}$, is reduced by more than 20% (more particularly more than 40 %), such that the remaining resting pressure $p_{rest2}$ is lower than 80% (more particularly lower than 60 %) as compared to the baseline resting pressure $p_{rest1}$, the evaluation unit 126 may indicate that compression is not effective any longer:

$$(P_{rest2}/p_{rest1}) \times 100\% < 80\%, \text{ preferably} < 60 \% \rightarrow \text{change compression}$$

As further indicated above, in this or other embodiments of the present invention, two or more key figures may be combined. As an example, absolute values of the resting pressure may be compared to one or more thresholds and, at the same time, a combination of two key figures may be compared to one or more thresholds. As an example, the evaluation unit 126 may be adapted to monitor that the actual resting pressure $p_{rest2}$ does not fall below 60 % compared to initial $p_{rest1}$ and, further, does not fall below an absolute pressure threshold of 15 mmHg. All threshold values described above may be combined this way.

B.) Measurement of the extended standing pressure $p_{standing, extended}$

As outlined above, the monitoring system 116 according to the present invention use the so-called extended standing pressure $p_{standing, extended}$ as a key figure for assessment of efficacy of the compression device 112. The extended standing pressure is measured by using a modified process of measuring the pressure in the upright standing position. Standing position can mean that the patient is standing on both feet without any movements. In the best case, the patient would have both hands holding on something to avoid muscle contractions for balancing. A more realistic, alternative approach would be that the patient is standing in elevated position (e.g. a step) on the non-investigational leg. The leg with the pressure sensor should not be moved and should hang without contact to the floor.

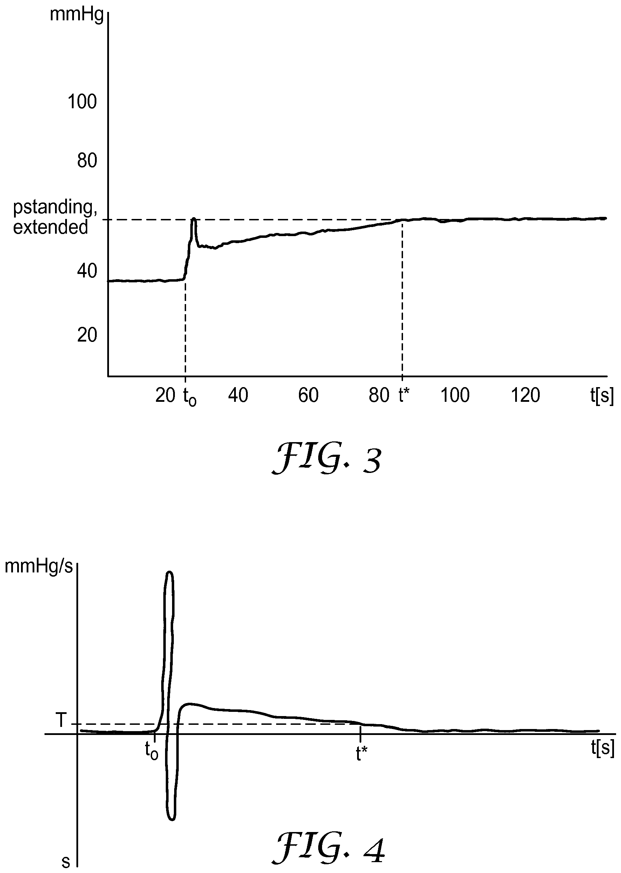

In Figure 3, a measurement curve of pressure values of a healthy volunteer is depicted. Initially, before the position change, a resting pressure $p_{rest1}$ of approximately 35 mmHg is detected. After the standing position, which takes place approximately at $t_0$, an overall increase in pressure compared to the resting pressure is observed. It will be appreciated that the move into the standing position typically causes a short, spike-like pressure peak(s) just after $t_0$ and any such pressure peak should be disregarded in determination of standing pressure. In Figure 3, just after $t_0$ the pressure peaks and subsequently thus falls to a value of approximately 48 mmHg, and thereafter, the pressure then increases as a result of venous filling to a pressure level of approximately 56 mmHg as an asymptotic value. Venous filling takes some time (approximately 40-90 s). In case of chronic venous disease including venous valve insufficiency, short refilling times will occur. The period of refilling time may also be used as a general criterion as will be described later.

As outlined above, if a compression device 112 becomes too loose because of material fatigue, volume reduction of the included leg, slippage of the system, or a combination, the system loses some of its capacity to keep the venous filling forces inside the compressed area. In other words, the system is less effective.

As can be seen in Figure 3, the pressure asymptotically approaches an asymptotic endpoint value due to venous filling. In conventional measurements of the standing pressure $p_{standing}$, the medical staff will simply measure the standing pressure at a predetermined point in time after the position change or at a point in time at which the measurement curve appears to have reached its endpoint value. This procedure, however, implies a specific irreproducible and subjective component. Therefore, according to the present invention, the extended standing pressure $p_{standing, extended}$ is determined. The extended standing pressure is typically measured in a stable upright position of the patient. After activation of the monitoring system 116, e.g. by pushbutton activation, the monitoring system may start to continuously acquire pressure values. Therein, optionally, as outlined above, averaging may take place, such as an averaging within time intervals of 1 second including 10 single measurements each. The average or preferably median of these 10 pressure values for each 1 second interval may be calculated continuously.

The evaluation unit 126 automatically evaluates the slope of the measurement curve and compares the slope to a predetermined threshold. In Figure 4, the slope of the measurement curve of Figure 3 is depicted. This slope simply may be the first derivative of the measurement curve of Figure 3. Therein, the vertical axis may denote the slope in arbitrary units, such as in mmHg per time. As soon as the slope in Figure 4 reaches a predetermined endpoint threshold value, denoted by T in Figure 4, the evaluation unit 126 may automatically recognize that an endpoint of change in the measurement curve has been reached. In Figure 4, this endpoint on the horizontal axis is denoted by t*. The pressure value in Figure 3, which is acquired at t* or after t*, is assigned to the extended standing pressure $p_{standing, extended}$.

As an example, in this embodiment or other embodiments, the endpoint threshold value T may be equal to or less than 0.5 mmHg per second, preferably equal or less than 0.05 mmHg per second, most preferably equal or less than 0.01 mmHg per second.

In the exemplary embodiment depicted in Figure 3, the median pressure differences of each 1 second time interval diminish when the measurement curve asymptotically levels out. If 5 consecutive time intervals show a pressure increase of less than 0.05 mmHg per second (which amounts to less than 0.25 mmHg per 5 time intervals) the system will store the standing pressure as the extended standing pressure $p_{standing, extended}$. As outlined under A, the median of the previous 5 time interval pressures may be stored as the extended standing pressure. All variations of criteria (e.g. length of time interval, number of single measurements) described under A are applicable for the measurement of the standing pressure also.

Also, the user could manually activate the monitoring system 116 by e.g. pushbutton to directly start measurement and documentation of the standing pressure. After completing measurement in the standing position, the system may provide an acoustical or optical signal. This signal may be also used to remind changing the position to walking as outlined later.

In this embodiment or any other embodiments, the extended standing pressure is used instead of the standing pressure as determined in a conventional way, such as for any subsequent evaluation of the efficacy of the compression system 110 of the compression device 112. The standing pressure as determined in a conventional way may, however, be used in addition, such as as an additional key figure.

Directly after application of the compression system 110, the standing pressure $p_{standing1}$ or the extended standing pressure $p_{standing, extended1}$, are usually higher than in every subsequent measurement ($p_{standing2}$, $p_{standing, extended2}$) performed at a later point in time. Initially $p_{standing1}$ and/or $p_{standing, extended1}$ should be higher than a predetermined threshold, such as 40 mmHg. Generally when using $p_{standing1}$ and/or $p_{standing, extended1}$ as a key figure, a threshold of 40 mmHg or lower may be used:

$$p_{standing1} \text{ and/or } p_{standing, extended1} < 40 \text{ mmHg (preferably} < 30 \text{ mmHg, more preferably} < 20 \text{ mmHg)} \rightarrow$$

change compression.

The measurement of the extended standing pressure and, optionally and additionally, the conventional standing pressure, may be repeated at a later point in time, such as after several minutes, several hours or even several days. As indicated above, the values derived thereby may be used as additional key figures and will be denoted as $p_{standing2}$ and $p_{standing, extended2}$ in the following. Again, these key figures may be compared to one or more threshold values.

Thus, as an example, the standing pressure $p_{standing2}$ and/or the extended standing pressure $P_{standing, extended2}$ should not fall below a threshold of 35 mmHg or lower:

$$p_{standing2} \text{ and/or } p_{standing, extended2} < 35 \text{ mmHg (preferably} < 25 \text{ mmHg, more preferably} < 15 \text{ mmHg)} \rightarrow$$

change compression

Both the extended standing pressure and the conventional standing pressure may be used for deriving further key figures for evaluating the efficacy of the compression device 112. Thus, as both the standing pressure and the extended standing pressure typically show inter-individual differences, a further option is to document the relative change of the actual $p_{standing2}$ and/or $p_{standing, extended2}$ in comparison to the initial (extended) standing pressure $p_{standing1}$ or $p_{standing, extended1}$, respectively, performed directly after application of the compression device 112. If $p_{standing2}$ and/or $p_{standing, extended2}$, respectively, is reduced by more than 20% (in particular more than 40 %), so that it is lower than 80% (in particular lower than 60 %) compared to baseline $p_{standing1}$ or $p_{standing, extended1}$, respectively, the evaluation unit 126 may indicate that the compression is not effective any longer.

Generally, in the present example or in other embodiments of the present invention, the threshold value for $p_{standing2}$ and/or $p_{standing, extended2}$ indicating inefficacy can be 80 % or preferably 60%:

$$(p_{standing2} / p_{standing1}) \times 100\% \text{ and/or } (p_{standing, extended2} / p_{standing, extended1}) \times 100\% < 80\%, \text{ preferably } < 60\% \rightarrow$$
change compression device

Also both, absolute and relative thresholds can be combined, e.g. the actual $p_{standmg(, extended)2}$ must not fall below 35 mmHg and the percent ratio of actual $p_{standmg(, extended)2}$ to the previous $p_{standmg(,extended)1}$ must not fall below 60%.

All threshold values described above can be combined this way.

The time period needed between changing from the resting position, such as the supine position, to the standing position of the patient until the time when the pressure does not further increase may also be used as a diagnostic criterion of chronic venous disease. Thus, in the example depicted in Figures 3 and 4 as well as in other examples, the time span from the position change ($t_0$) to the endpoint ($t^*$) may be used as a further key figure, indicating a refilling time $t_{refill} = t^* - t_0$. Especially functional changes due to valve insufficiency and venous ectasia (dilatation) may be judged by this time interval. Venous refilling also may change from baseline to follow up measurements. Also different compression systems might have varying refilling times.

The more compression a system delivers to the extremity, the more these forces counteract venous dilatation and consecutively valve insufficiency. This positive effect to the venous system can lead to longer refilling time. The refilling time could hence also be used to assess how effective the compression system influences venous reflux.

In this example or in other examples of the present invention, when using the refilling time as a key figure, the refilling time ($t_{refill}$) may be measured more than once, at different points in time. Thus, as an example, the refilling time may be measured at baseline and at follow-up. Differences between baseline and follow-up, again, may be compared to one or more threshold values. Thus, as an example, the difference of less than 5 seconds between refilling times measured at different points in time may be considered optimal, while differences in refilling times of greater than five seconds, more particularly greater than 10 seconds may be considered as an indication to change the compression system

$$t_{refill1} - t_{refill2} > 5 \text{ s (preferably} > 10 \text{ s)} \rightarrow \text{change compression}$$

Furthermore, a refilling time which may be overall too short may indicate a venous valve insufficiency and could be an indication for a warning for the user to consult with the medical practitioner. Thus, as an example, as an upper threshold value for the absolute refilling time may be 30 seconds:

$$t_{refill} < 30 \text{ s} \rightarrow \text{warning signal (short refilling time/consult medical practitioner)}$$

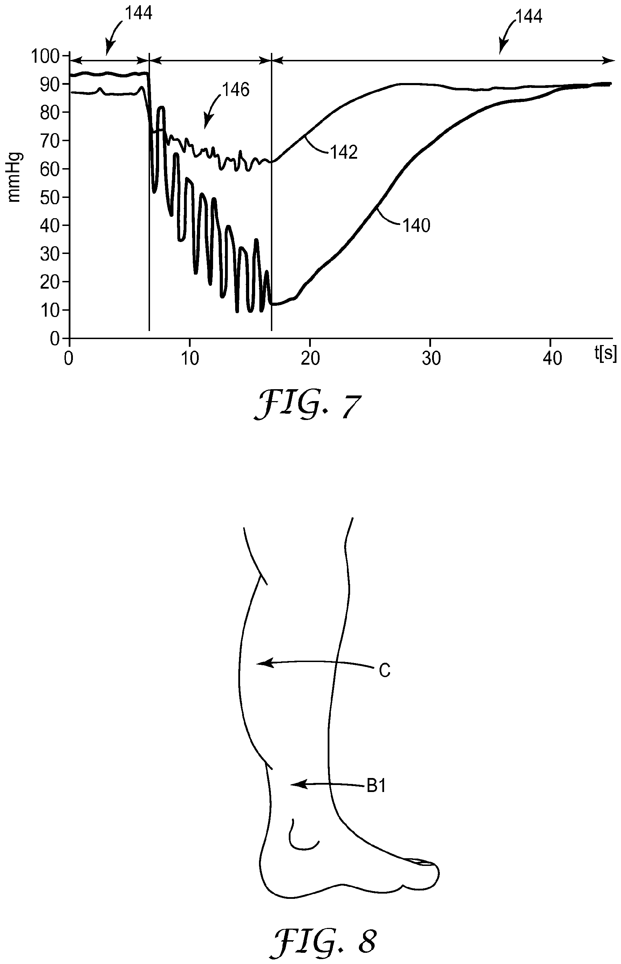

In Figure 7, measurement curves 140 and 142 are depicted, wherein measurement curve 140 shows a measurement curve of a normal limb, whereas measurement curve 142 shows a measurement curve of a limb with incompetent venous valves. Therein, time spans 144 denote periods in which the person is standing, whereas time span 146 denotes a period in which the person is walking. As can be seen, the refilling time for the limb with incompetent venous valves, the refilling time is significantly shorter than for the limb with normal venous valves.

Beside the venous refilling time, also the shape of the measurement curves, such as the measurement curves 140 and 142 in Figure 7 or the measurement curve in Figure 3, can act as a key figure and may provide information about the anatomical fit of the compression device 112 and its stiffness. With good anatomic fit, the increase of pressure typically is moderate directly after position change to standing. In this phase, the initial increase of volume does not find very strong counter bearing due to the compression system. With further volume gain, the tensile elastic limit is advanced and hence the pressure curve becomes steeper until the increase alleviates and the curve approximates to the asymptote. A slightly sigmoidal shape of the curve is typical for a good anatomic fit and sufficient stiffness of the compression device 112.

C.) Measurement of static stiffness

Further, a significant key figure for evaluating the efficacy of the compression device 112 may be the so-called static stiffness index SSI. Again, the static stiffness index, which is generally known in the art, may be calculated by using the conventional standing pressure and/or by using the extended standing pressure, as discussed above. In case the conventional standing pressure is used, the expression "SSI" will be used in the following, whereas, in case the extended standing pressure is used, the expression "ESSI" (extended static stiffness index) will be used in the following.

The static stiffness index generally denotes the difference between the pressure in the resting position, such as in the supine position, and the pressure in the upright position. For effective compression of chronic venous insufficiency and leg ulcer, high stiffness is considered to be most effective. After some days of application of the compression device 112, such as after some days after application of a compression bandage, the SSI (or ESSI, respectively) may have changed in comparison to the baseline status directly after application of the compression device 112. This effect may be caused by material fatigue, slippage of the bandage or the therapeutic effect of limb volume reduction.

For assessing the SSI or ESSI, respectively, the sub-bandage pressure may be measured in the resting position first, by measuring as explained above in section A. For assessing the standing pressure $p_{standing}$ or the extended standing pressure $p_{standing, extended}$, reference may be made to section B above.

After the monitoring system 116 has finished measuring the resting pressure, an acoustical signal may be given. Additionally or alternatively, other invitations for changing position may be provided to the user. After this signal, the patient should change to the standing position. As indicated above, after some time, the pressure signal becomes stable and the evaluation unit 126 may automatically detect the extended standing pressure as described in section B above. Additionally or alternatively, as explained above, a conventional method may be used for measuring the standing pressure.

Instead of using actual measurements for determining the static stiffness index and/or the extended static stiffness index, additionally or alternatively, values provided by data input may be used. Thus, another procedure may be to enter the information of the patient's position via a pushbutton, keypad or touch screen. After the system gets this information of position change, the system continues to measure pressure in time intervals for evaluation of the standing pressure as described in section B above .

Generally, the evaluation unit 126 may document two pressures, one in resting position, one in standing position.

The static stiffness index (SSI) may be defined by the following formula:

$$SSI = p_{standing} \, [mmHg] - p_{rest} \, [mmHg]$$

Similarly, the extended static stiffness index (ESSI) may be defined by:

$$ESSI = p_{standing, extended} \, [mmHg] - p_{rest} \, [mmHg]$$

Several parameters may have an influence on the SSI or ESSI, respectively. Thus, SSI and/or ESSI may be related to a bandage material, the degree of bandage stretch when applied by the therapist, a size and an activity of the muscles such as the calf musculature or the mobility of certain joints, such as the mobility in ankle joints, especially in elderly patients and it may be related to the location on the limb, where the pressure is measured, (see figure 8). Over time of bandage application, the SSI or ESSI, respectively, may change indicating that the compression device 112, such as the compression bandage, is not effective any more.

In the above-mentioned publication by Mosti et al., some experimental results are disclosed, which compare measurements taken immediately after the application of the compression device 112 and measurements taken one week after application, for a plurality of 100 patients. The data reveal that effective ulcer healing correlates with a SSI that does not drop significantly over time. By using the extended static stiffness index ESSI instead of the conventional SSI, as proposed herein, the reproducibility and precision of the key figure SSI / ESSI may further be increased.

Again, in this example or in other exemplary embodiments of the present invention, the key figure of the extended static stiffness index again may be compared to one or more threshold values. Thus, generally, as for all other key figures, the evaluation unit 126 may be adapted to perform this comparison automatically. Again, the key figures may be determined repeatedly at different points in time, such as immediately after application of the compression device 112 as well as after a certain time span after application of the compression device 112, such as after several minutes, several hours or even several days. As an example, a lower threshold for the initial static stiffness index and/or for the initial extended static stiffness index selected. Thus, as an example, directly after application of the compression device 112 such as the compression bandage, the initial SSI ($SSI_1$) and/or the initial ESSI ($ESSI_1$) may be assessed by the monitoring system 116, such as by the evaluation unit 126. The evaluation unit 126 may be programmed to indicate inefficacy of the compression device 112, if the $SSI_1$ and/or the $ESSI_1$ is lower than a select threshold of e.g. 10 mmHg or 15 mmHg, respectively (or preferably an even lower threshold of 5 mmHg or 10 mmHg, respectively):

$$SSI_1 = p_{standing1} \, [mmHg] - p_{rest1} \, [mmHg]$$

$$SSI_1 = p_{standing1} \text{ [mmHg]} - p_{rest1} \text{ [mmHg]}$$

$$SSI_1 < 10 \text{ mmHg (preferably} < 5 \text{ mmHg)} \rightarrow \text{change compression}$$

And/or:

$$ESSI_1 = p_{standing, extended1} \text{ [mmHg]} - p_{rest1} \text{ [mmHg]}$$

$$ESSI_1 < 15 \text{ mmHg (preferably} < 10 \text{ mmHg)} \rightarrow \text{change compression}$$

As indicated above and as valid for any key figure K used for assessment of the efficacy of the compression device 112, the key figure of the static stiffness index and/or the key figure of the extended static stiffness index may be determined repeatedly, such as by determining this key figure at a later point in time. Thus, for control of effective compression, a subsequent measurement of the SSI and/or ESSI may be performed at a later in time point.

Again, as valid for any type of key figure, the key figure determined at a later point in time again may be compared to one or more threshold values which may be different from the threshold values applied to the previously determined key figures. Additionally or alternatively, the key figure determined at a later point in time may be compared to the respective key figure previously determined.

Thus, as an example, the monitoring system 116 and, specifically, the evaluation unit 126, may indicate inefficacy in case the $SSI_2$ and/or the $ESSI_2$ are below a predetermined threshold value. Generally, in this embodiment or other embodiments, this threshold value may be lower than those described for $SSI_1$ and $ESSI_1$. As an example, the following comparisons may be performed by the evaluation unit 126:

$$SSI_2 = p_{standing2} \text{ [mmHg]} - p_{rest2} \text{ [mmHg]}$$

$$SSI_2 < 5 \text{ mmHg (preferably} < 3 \text{ mmHg)} \rightarrow \text{change compression}$$

And/or:

$$ESSI_2 = p_{standing, extended2} \text{ [mmHg]} - p_{rest2} \text{ [mmHg]}$$

$$ESSI_2 < 10 \text{ mmHg (preferably} < 4 \text{ mmHg)} \rightarrow \text{change compression}$$

Again, as the SSI and/or EESI typically show inter-individual variations, a further option may be to add the relative change of the actual $SSI_2$ and/or $ESSI_2$ in comparison to the initial $SSI_1$ or $ESSI_1$, respectively, the latter acquired directly after application of the compression device 112, such as directly after bandage application. If the actual $SSI_2$ and/or $ESSI_2$ is reduced by more than a predetermined threshold, such as 20%, preferably 40 %, as compared to the initial value $SSI_1$ or $ESSI_1$, respectively, the monitoring system 116 and, specifically, the evaluation unit 126 may indicate that the compression device 112 is not effective any longer.

As an example:

$$SSI_2 = p_{standing2} \text{ [mmHg]} - p_{rest2} \text{ [mmHg]}$$

$$SSI_1 = p_{standing1} \text{ [mmHg]} - p_{rest1} \text{ [mmHg]}$$

$$(SSI_2 / SSI_1) \text{ x } 100\% < 80 \text{ \%, preferably} < 60 \text{ \%} \rightarrow \text{change compression}$$

And/or:

$$\text{ESSI}_2 = p_{\text{standing, extended2}} \, [\text{mmHg}] - p_{\text{rest2}} \, [\text{mmHg}]$$

$$\text{ESSI}_1 = p_{\text{standing, extended1}} \, [\text{mmHg}] - p_{\text{rest1}} \, [\text{mmHg}]$$

$$(\text{ESSI}_2 : \text{ESSI}_1) \times 100\% < 80\%, \text{ preferably} < 60\% \rightarrow \text{change compression}$$

Again, as for all the key figures, absolute and relative thresholds may be used and/or may be combined. Thus, as an example, the actual $\text{SSI}_2$ and/or $\text{ESSI}_2$ may be monitored in order not fall below 60 % compared to initial $\text{SSI}_1$ or $\text{ESSI}_1$, respectively, and also may be monitored in order not fall below an absolute value of 5 mmHg or 10 mmHg, respectively. All threshold values described above may be combined this way.

D.) Measurement of amplitudes

As outlined above, one or more amplitudes of measurement curves during a defined activity or movement, such as walking, of the user may be used as one or more additional key figures for determining the efficacy of the compression device 112.

Thus, as an example, due to calf muscle contraction within a rigid sleeve, mainly by the musculus gastrocnemius and soleus, the sub-bandage pressure typically shows short termed pressure peaks. These amplitudes, generally defined by the differences between the pressure values in the upper and lower pressure peaks in the measurement curve, may be used as another key figure and, thus, as another option for evaluating the efficacy of a compression device 112. Again, this key figure may provide an indication of how well an applied compression system 110 manages to keep forces produced by the muscle activity inside the compressed area.

In Figure 5, an example of a measurement curve acquired during a controlled movement of the user is depicted. In this figure, a period of resting (such as in the supine position) is denoted by reference number 148, whereas, as in Figure 7, periods of standing and periods of walking are denoted by reference numbers 144 and 146, respectively. In this measurement as depicted in Figure 5, the pressure sensor 118 was placed in a position denoted by B1 in Figure 8, which shows a lower leg of the user. Instead of walking, any other type of controlled functional activity and/or exercise may be used.

As an example, for performing the measurement, the patient has to be in the upright position and has to walk on a belt or has to do other "defined" physical activities. Due to calf muscle contraction, the sub-bandage pressure shortly increases and immediately decreases again within the diastolic phase of muscle relaxation.

The monitoring system 116, specifically the evaluation unit 126, may automatically detect specific activities. Thus, the evaluation unit 106 may automatically detect that the patient is walking on a belt or stepper. See portion of the curve with its alternating pressure curve marked with the reference number 146 in Figure 5. Thus, within the phasic curve as shown in Figure 5, the evaluation unit 126 may detect Amplitude as key figure.

When monitoring amplitudes, the amplitudes may be evaluated statistically. A median or mean value may be formed and compared to one or more threshold values. Thus, as an example, if a median or mean amplitude is below a predetermined threshold of e.g. 40 mmHg, more particularly of 15 mmHg, the system may indicate that compression is not effective any more:

$$\text{Amplitude}_{\text{median}} \text{ or Amplitude}_{\text{mean}} < 40 \text{ mmHg (preferably} < 15 \text{ mmHg)} \rightarrow \text{change compression}$$

Median or mean amplitudes measured at different points in time may be compared. Thus, again, a ratio of these amplitudes may be formed and may be compared to one or more threshold values. As an example, the monitoring system 116 may indicate an inefficacy of the compression device 112 in case $\text{Amplitude}_{\text{median2 or mean2}}$ is less than 80% (preferably less than 60 %), as compared to $\text{Amplitude}_{\text{median1 or mean1}}$:

$$(\text{Amplitude}_{\text{median2}}/ \text{Amplitude}_{\text{median1}}) \times 100\% \text{ or } (\text{Amplitude}_{\text{mean2}}/ \text{Amplitude}_{\text{mean1}}) \times 100\% < 80\% \text{ (preferably } 60\%) \rightarrow \text{change compression}$$

Also both, absolute and relative thresholds may be combined, e.g. the actual median amplitude must not fall below 60 % compared to the initial amplitude and also must not fall below 15 mmHg. All threshold values described above may be combined this way.

<u>E.) Multiparameter measurement of pressure values</u>

A further method to assess efficacy of a compression system is to combine two or more key figures, such as two or more of the key figures listed above in sections A-D. Thus, the determination of each key figure, such as the key figures of sections A-D above, may be used as a single module for measurement. Additionally or alternatively, an arbitrary combination of key figures may be possible, which may lead to a multi-parameter assessment. A multi-parameter assessment may allow for a more precise and more reproducible assessment of a sub-bandage pressure profile.

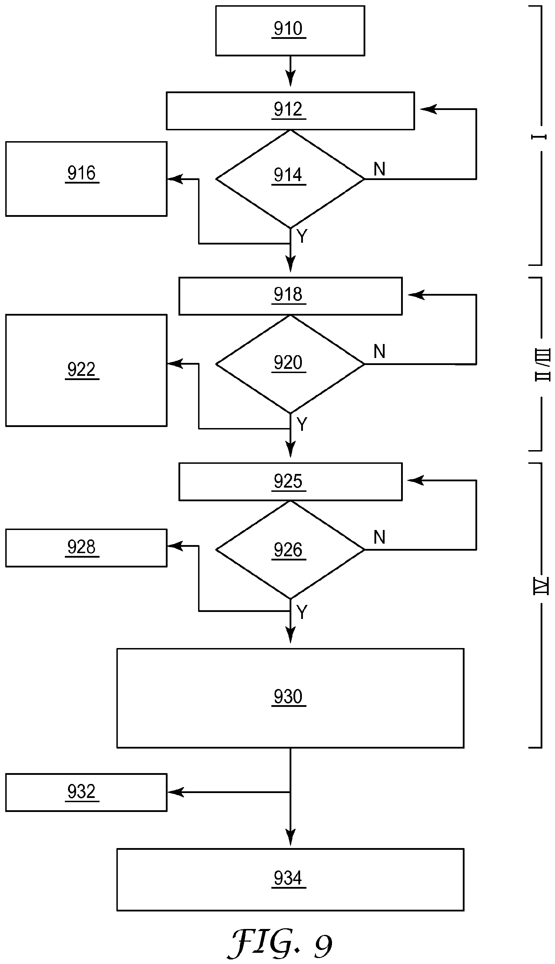

An example of a method using a multi-parameter assessment is depicted in Figure 9. Therein, several (in this case four) consecutive measurement modules are used:

| | |
|---|---|
| Module I: | According to section A above, the resting pressure is measured. |
| Module II: | According to section B above, the standing pressure $p_{standing}$ and/or the extended standing pressure $p_{standing}$, extended is measured. |
| Module III: | According to section C above, the SSI and/or ESSI is measured based on the resting and the standing pressure or on the resting and the extended standing pressure, respectively. |
| Module IV: | According to section D above, the working pressure amplitudes are measured. |

It shall be noted that any other combination of key figures is possible. Thus, an arbitrary combination of the modules I to IV above may be used.

In Figure 9, the following method steps may be used:

| | |
|---|---|
| 910 | Start by push button |
| 912 | Measurement of resting pressure |
| 914 | Query: Stable resting pressures? (Y: Yes, N: No) |
| 916 | Display result $p_{rest}$ and display "change position to standing position" |
| 918 | Measurement of standing pressure $p_{standing}$ and/or measurement of extended standing pressure $P_{standing}$, extended |
| 920 | Query: Stable standing pressure? (Y: Yes, N: No) |
| 922 | Display result $p_{standing}$ and/or $p_{standing}$, extended, calculate and display SSI and/or ESSI, display "change to walking" |
| 924 | Measurement of amplitude |
| 926 | Query: Stable amplitude? (Y: Yes, N: No) |
| 928 | Determine amplitude and optionally display result |
| 930 | Calculation of status (Baseline / Baseline versus Follow-up) Calculation of key figures (e.g. SSI, ESSI, etc.) Analysis of key figures (modules I-IV) |
| 932 | Display of results, e.g. status, key results, whether compression device should be changed (the latter could be done in the form of a traffic light, e.g. red light change compression device, green status is good and yellow warning) |
| 934 | Store results, e.g. key values, status information, figures, etc. values, figure, key values |

For details of these steps, reference may be made to the disclosure of the single modules above. Although not specifically depicted in Figure 9, in case a multiple (e.g. two or more) of NO answers to any one of the queries under 914, 920, 926 occurs, to avoid a continuous looping at that point, the method can be arranged such that after a certain select number of NO answers (e.g. two or three) a routing is provided directly down to 932 so that the issue (e.g. unstable resting pressure measurement) can be displayed.

<u>Interface between algorithm and the evaluation unit 126 and/or pressure sensor 118</u>

In this embodiment or in other embodiments of the present invention, the pressure sensor 118 or, in case a plurality of pressure sensors 118 is used, each of the pressure sensors 118 may comprise an electronic identifier, such as a contactless electronic identifier, which allows for a unique identification of the pressure values provided by the respective pressure sensor 118. As an example, a RFID may be used as an electronic identifier.

The electronic identifier, such as the RFID, within the pressure 118 sensor may be activated by first readout of the reader. Thus, the evaluation unit 126 may comprise a reader for reading out the electronic identifier.

The monitoring system 116 may further be adapted to automatically detect new components, such as newly implemented pressure sensors 118. If the monitoring system 116 detects a new electronic identifier, such as a new RFID, the monitoring system 116 may save all measured values as the baseline status. Subsequent measurements which may be repeated within a predetermined time span, such as within 3 hours after first activation, may overwrite the first baseline values. This procedure may allow for repeating false baseline measurements. The predetermined time span, such as the period of 3 h, may as well be chosen shorter or longer than 3 h, such as 10 minutes up to 24 h.

After a waiting time according to the predetermined time span, such as after a period of 3 h, any following measurements may be stored as follow-up assessments for the compression system 110. By using the electronic identifier, such as by using the RFID, the monitoring system 116, specifically the reader, may automatically assign the follow-up values to the right patient and all subsequent values will be compared to the appropriate baseline values. Due to this procedure, deletion of data by mistake may be excluded.

Further, a mixing up of patient data may be avoided by using electronic identifiers. Thus, a reuse of the sensor electronics may be avoided in order to avoid false RFID assignment to another patient, which may lead to incorrect calculation of baseline versus follow-up data.

In the method depicted in Figure 9, various options exist for combining the phases and/or for evaluating the phases. Several options will be given in the following:

Option 1:

Each phase can be assessed separately. So the resting pressure, standing pressure, extended standing pressure, the SSI, the ESSI and the amplitude may each be used as a single measurement. A button may be used to get into the right mode of compression measurement (e.g. resting pressure).

Option 2:

Combinations including a fixed sequence of measurements as shown in the flow-chart may be used to receive a more comprehensive picture of the actual properties of the compression device 112.

For this purpose, a start button may be activated to start the measurement. The resting pressure may be assessed and documented automatically as described in section A above. An acoustical and/or numerical signal may provide information that the monitoring system 116 has completed the first measurement in resting position. If the measurement should be repeated immediately (e.g. due to false position of the patient), a ("start") button may be pushed again.

An acoustic signal may invite the user or patient to change into the standing position. As described in section B above, the device may automatically detect the accurate standing pressure. An acoustical and/or numerical signal may provide information that the monitoring system 116 has completed the second measurement in the standing position. If the measurement should be repeated immediately (e.g. because the patient has been moving excessively), a "start" button may be pushed again.

On the basis of the resting pressure and the standing pressure or the resting pressure and the extended standing pressure, the monitoring system 116 may automatically calculate the third parameter SSI and/or ESSI, respectively, as disclosed above in section C.

After an appropriate invitation by the monitoring system 116, such as after an acoustic signal, the patient may start to walk on a treadmill, stepper or another device that allows continuous and periodic exercise, preferably in a controlled and reproducible way. As described in section D above, the monitoring system 116 may automatically measure the working pressure amplitude. An acoustical and/or numerical signal may provide information that the monitoring system 116 has completed the measurement in the walking position. If the measurement should be repeated immediately (e.g. because the patient did not walk regularly), a "start" button may be pushed.

Finally, the monitoring system 116 may either display all values numerically, including the resting pressure, the standing pressure, the extended standing pressure, the SSI, the ESSI, the amplitude, or any arbitrary combination thereof, as well as, optionally, appropriate changes with reference to the respective baselines, such as the percentage changes compared to baseline. Additionally or alternatively, the monitoring system 116 may automatically calculate if the compression device 112 is still effective, such as by evaluating one or more key figures, e.g. according to one or more of the algorithms and/or threshold values disclosed in sections A-D above. If these calculations are based on more than one value, the thresholds given under A-D might change within the given ranges.

Generally, in this embodiment or other embodiments, an information regarding the efficacy of the compression device 112 (such as whether the compression device 112 is still effective or not) may be provided to the user in an arbitrary way, such as by visual display. As an example, a "traffic light" type display may be used, indicating an efficacy by a green light, an intermediate or reduced efficacy by a yellow light, and an inefficacy by a red light.

F.) Measurement of arterial pulsations

As outlined above, specifically with respect to Figure 6, one or more key figures derived from the detection of arterial pulsations may be used for determining the efficacy of the compression device 112. Thus, generally, sufficient arterial perfusion is a prerequisite for adequate tissue metabolism and healing processes in patients with chronic leg ulcer. Compression with too high resting pressure may cause arterial under-perfusion and may cause delayed or interrupted wound healing. According to Pascal's law, pressure typically is equally distributed in tissues. This generally means that volume changes synchronous to arterial pulsation can be measured under a stiff compression device 112. In case the pulsation is recognized by the monitoring system 116, arterial macro-perfusion is likely to be existent under the compression device 112. This information can be valuable for safety reasons especially in patients with arterial perfusion disorders.

Thus, in addition or alternatively to some of the key figures disclosed in sections A-E above, one or more key figures derived from arterial pulsations may be used. Thus, periodic oscillations in one or more of the measurement curves may be detected, preferably over the whole period of measurement time. Periodic oscillations due to arterial pulsations (denoted by reference number 136 in Figure 6) typically are to be expected within a frequency band of 0.7 to 1.8 Hz. The frequency band can also be wider with 0.5 up to 2.5 Hz. As outlined above, electronic filters and/or mathematical evaluation means may be used for detecting the arterial pulsations within the measurement curves, such as Fourier analysis. Thereby, the frequencies of pulse, breathing (respiratory activity 138 in Figure 6) and other periodic loads (e.g. Walking, see section D above) may be determined and may be distinguished from arterial pulsations.

G.) Assessment of patient activity profile

During position changes of the leg, walking or training exercise, sub-bandage pressure typically changes and venous blood is consecutively shifted in proximal direction back to the central circulation. Typically, one important aspect of sufficient and appropriate compression therapy is the cooperation of the patient. Physical exercise, walking, biking or in minimum some movement, increases the venous flow under compression therapy.

Generally, by using the monitoring system 116 having the at least one pressure sensor 118, an activity profile of the patient may be recorded and may be evaluated. Thus, two or more intensity levels may be identified in one or more continuous measurement curves of pressure values provided by the pressure sensor 118, wherein, for example, for each intensity level of the activity profile, the monitoring system 116 may evaluate how much time the patient has spanned within the respective intensity level.

Generally, an algorithm may be used which is capable of finding predefined pressure alterations which are typically observed under movement. The algorithm may be capable of detecting pressure alterations, here defined as Exercise Events (EE). An EE is defined as an absolute (positive or negative) change of pressure larger than 1-30 mmHg, preferably 5 mmHg. This pressure alteration should occur within a time period of 0.1-10 s, preferably 1 s. EEs may be recorded over the whole time of application of the compression device.

Over one hour or up to one or more days, the amount of EEs per time period (e.g. 1 hour) may be calculated and rated on an activity index, such as on a 1-10 Activity Index (AI) scale. A low AI means no or low activity, a higher Index means that the patient sufficiently moved and consecutively supported the clinical benefit of the compression system. The scale for AIs can be larger with up to 1-100 for more precise differentiation of activity intensities.

Further, EEs with varying intensities may be distinguished, e.g. $EE_1$ with $\geq$ 3-6 mmHg, $EE_2$ > 6-10, $EE_3$ with > 10 mmHg absolute pressure difference:

$$EE_1 \geq \left| \text{3-6 mmHg} \right|$$

$$EE_2 > \left| \text{6-10 mmHg} \right|$$

$$EE_3 > \left| \text{10 mmHg} \right|$$

EEs with different intensities may also be weighted, so that one $EE_3$ has more impact than one $EE_1$ for example:

$$\text{Impact EE1} < \text{Impact EE2} < \text{Impact EE3}$$

Instead of 3 intensity levels, a different number of intensity levels may be used. Thus, also 2-100 EEs can be defined for more precise activity evaluation.

Further, other key figures may be used in addition. Thus, one or more of the key figures $SSI_1$, $SSI_2$, $ESSI_1$, $ESSI_2$ and amplitudes, measured according to sections C and D above, may be used to adjust the varying intensities of the EEs. This procedure can be helpful as the working amplitudes may decrease due to material fatigue over time of wearing albeit the patient exercised with equal intensity.

The allocation of the patient activity to a value of the AI scale (e.g. 4 on a 1-10 scale) can be predefined by the monitoring system 116. Further, the therapist may adjust this AI allocation according to the physical condition of the patient. For example, a patient with a significant walking disability may have the same definition for EEs to maintain comparability. However, the AI scale can be less stringent to maintain enough resolution even for low activity profiles.

In parallel to the Activity Index generated by pressure gradients, also a motion sensor placed on the leg, foot or other parts of the body may be added to the monitoring system 116. This motion sensor may be capable of tracking continuous information about movements. This information can be used to complete the AI profile. Also, the motion sensor can be used to activate the "sleep" modus in case no activity is detected. In this case, the interval from one single measurement to the next measurement will be increased to prolong the life of a battery.

Patient coaching

The description above summarizes how the activity profile may be recorded to allow the therapist appropriate medical judgment and consecutive instructions for the patient. In a further step, the monitoring system 116 could also coach the patient to achieve good physical activity for optimal compression effects.

For this purpose, the therapist might feed the system with a minimum required AI rate for a predefined time interval. With e.g. an acoustical signal, the monitoring system 116 may confirm acceptable activity, or in opposite demand further movement to optimize the action of the compression system. With a green, yellow, or red light or a smiley, the patient may be informed about the current activity achievement.

Further, as outlined above, a motion sensor could add information about the activity profile of the patient.

H.) Continuous safety surveillance system of critical overpressure

Typically, a high pressure exerted by the compression device 112 is rather uncritical, as long as the overpressure lasts for a short period only. This is typically observed if patients walk or do other physical activity. However, if the pressure is continuously high, e.g. in the supine position at night, there is a risk for pressure related skin damage. For safety reasons it is therefore useful to optionally provide a warning in case the pressure exceeds a defined threshold value, such as for a longer time period.

As an example of a safety surveillance system which may be implemented into the monitoring system 116, the pressure may be recorded automatically. High pressure may be defined as a pressure exceeding a predetermined threshold, such as a pressure exceeding a threshold of 60 mmHg, preferably 80 mmHg, most preferably 100 mmHg. A warning may be created by the monitoring system 116 in case the pressure exceeds the predetermined threshold, such as for at least a predetermined time period. Thus, as an example, in case the pressure is continuously higher than e.g. 80 mmHg over a period of more than 1 s, preferably 120 s, most preferably 600 s, the monitoring system 116 may provide a warning, such as by an output of an acoustic signal and/or a visual signal. In such a case, the patient should change the position or walk, or move toes or the limb. In many cases, this can already change the applied forces exerted by the compression device 112. In a worst case, if changing the body position of movement does not help, the patient may have to remove the compression device 112, such as the compression bandage, or may have to reduce the tension in case of an adjustable compression system 110.

In case of a coincident disease, e.g. peripheral arterial occlusive disease, pressure may be more critical. In this case, the therapist may adjust the threshold for pressure and the time of pressure according to the patient's needs.

I.) Continuous surveillance of insufficient pressure profiles

As outlined above, resting and standing pressure (including extended standing pressure) as well as pressure amplitudes may be measured, such as by a nurse, a physician, a therapist or any other medical staff. For this procedure, the patient may be instructed to change to the needed body position.

In order to allow for an assessment of compression efficacy independently from any therapist or clinical visit, the monitoring system 116 may also continuously monitor the pressure profiles and, hence, the efficacy of the compression device 112. Therein, various options exist. Several potential options are described below:

Option 1: Once a day (such as 1-20 times a day) the patient may initiate a measurement, such as by pressing a button on the monitoring system 116, and will assume a resting position, such as by assuming a supine position, as described in section A above. The monitoring system 116 may automatically measure the resting pressure, such as once the measurement curve has stabilized. Subsequently, the evaluation unit 126 may invite the patient to change a position. Thus, an acoustic signal may be provided to the patient. The patient may then change into the standing position, and the monitoring system 116 will again measure the pressure, preferably automatically. The results of $p_{rest2}$ and $p_{standing2}$ and/or $p_{rest2}$ and $p_{standing, extended2}$ may be compared to the initial data $p_{rest1}$ and $p_{standing1}$ and/or $p_{rest1}$ and

$p_{standing, extended1}$, preferably automatically. A difference between the baseline ($p_{rest1}$ and $p_{standing1}$ and/or $p_{rest1}$ and $p_{standing, extended1}$) and follow-up measurements ($p_{rest2}$ and $p_{standing2}$ and/or $p_{rest2}$ and $p_{standing, extended2}$) may be processed, such as disclosed above in sections A and B.

The same procedure may be performed with one or more of the SSI, the ESSI and the pressure amplitude, as disclosed in sections C above and D.

As previously discussed, the monitoring system 116 may indicate if the compression device 112, such as the compression bandage, is not effective any more.

Option 2: Once a day (such as 1-20 times a day) the monitoring system 116 may provide an invitation to the patient, such as by providing an acoustic signal. After that, the patient may change to a resting position, such as to the supine position, and, later on, to the standing and/or walking position as described above under 1.

Option 3: For permanent assessment of the efficacy of the compression device 112, the monitoring system 116 may continuously measure the pressure. The monitoring system 116 may acquire measurement curves and may detect the standing pressure and/or the extended standing pressure, such as by evaluating the asymptotic behavior of the measurement curve, as disclosed above in sections A and B. An asymptotic function typically is only detected if the patient is at rest, e.g. in supine or sitting position, and/or if the patient is standing without significant movement. In the supine position, the lowest pressure curves are expected.

By detecting the lowest pressure value in the measurement curve, and, further, by assuming that this lowest pressure value is measured in a resting position, specifically in a supine position, the lowest pressure value may be recorded. Thus, as an example, the lowest pressure value acquired within 1 h up to 1 day, preferably 12 h, may be recorded as the actual resting pressure $p_{min}$. In parallel, the same procedure optionally may be performed with the maximum asymptotic pressure curve. This value may be recorded as $p_{max}$. The difference of $p_{min}$ and $p_{max}$ may be defined as $\Delta p$:

$$p_{max} - p_{min} = \Delta p$$

$\Delta p$ typically only provides a very rough approximation of the SSI or ESSI, respectively. It may be desirable to compare a $\Delta p$ of the first day ($\Delta p_1$) with a $\Delta p$ of the second day ($\Delta p_2$). The monitoring system 116 may indicate inefficacy, if the difference between $\Delta p_1$ and $\Delta p_2$ is greater than a predetermined threshold, such as 3 mmHg, more preferably 10 mmHg:

$$\Delta p_1 - \Delta p_2 > 3 \text{ mmHg (preferably} > 10 \text{ mmHg)} \rightarrow \text{change compression}$$

Further, additionally or alternatively, relative changes may be used to define inefficacy. Thus, pressure changes $\Delta p_1$ and $\Delta p_2$ measured at different points in time may be compared. Thus, again, a ratio of these pressure changes may be formed and may be compared to one or more threshold values. As an example, the monitoring system 116 may indicate an inefficacy of the compression device 112 in case $\Delta p_2$ is less than 80% (preferably less than 60 %), as compared to $\Delta p_1$:

$$(\Delta p_2/\Delta p_1) \times 100\% < 80 \%, \text{ preferably} < 60 \% \rightarrow \text{change compression}$$

Option 4: A further optional method for assessment if the compression device 112 is still effective may be the assessment of amplitudes when the patient performed a particular activity, such as walking, as described in detail above in Section D.

Position of the pressure sensor 118

A single pressure sensor 118 may be applied at the medial aspect of the lower leg, at the transition of the gastrocnemius muscle into the Achilles tendon. This position is denoted by B1 in Figure 8 and is situated typically approximately 10-15 cm proximal to the medial malleolus.

As this point covers only a small anatomical area, it is easy to imagine that a pressure sensor 118 may easily be misplaced.

Positioning a pressure sensor 118 on the muscular part of the calf (position C in Figure 8) is less sensitive. Therefore, another option is to position the pressure sensor 118 on the calf muscles. Threshold values as provided in sections A-D above might be changed in accordance to the actual placement of the pressure sensor 118 and/or in accordance with the anatomical area in which the pressure sensor 118 is placed. The above-mentioned thresholds, however, are preferred for the B1 position.

Also a plurality of pressure sensors 118 may be used in order to assess pressure at several areas. Further, one or more large area pressure sensors 118 might be used. Thus, as an example, one big wide pressure sensor 118 may be used which covers the whole leg. This pressure sensor 118 might be capable of measuring the pressure under relevant portions or even under the whole surface of the compression device 112.

The at least one pressure sensor 118 and/or the pressure sensor positions defined above may be used for all described methods to measure compression efficacy.

**Claims**

1. A monitoring system (116) for determining the efficacy of at least one compression device (112) for use in compression therapy, the monitoring system (116) comprising:

   - at least one pressure sensor (118) for measuring a pressure exerted onto a body part of a user by the compression device (112);
   - at least one attitude sensor (122) for acquiring at least one attitude information on at least one of a position, an orientation and a movement of the user;
   - at least one measuring device (120) having at least one evaluation unit (126), wherein the measuring device (120) is adapted to communicate with the at least one pressure sensor (118) and the at least one attitude sensor (122), wherein the at least one evaluation unit (126) is adapted to receive at least one pressure value acquired by the at least one pressure sensor (118) and wherein the at least one evaluation unit (126) is adapted to receive at least one attitude information acquired by the at least one attitude sensor (122);

   wherein the at least one evaluation unit (126) is adapted to automatically combine the at least one pressure value and the at least one attitude information in order to determine at least one key figure K indicating the efficacy of the compression device (112) taking into account the at least one attitude information, wherein the at least one evaluation unit (126) is adapted to perform a real-time determination of the key figure; and
   **characterized in that** the evaluation unit (126) is further adapted to perform the following procedure for determining an extended standing pressure $p_{standing, extended}$:
   acquiring a measurement curve of pressure values after a position change of the user into the standing position; automatically comparing a slope of the measurement curve to at least one endpoint threshold value and, depending on a result of the comparison, automatically detecting an endpoint of a change in the measurement curve induced by the position change, and assigning a pressure value acquired at or after the endpoint to the extended standing pressure $P_{standing, extended}$; and **in that** the at least one key figure is selected from the group consisting of:

   - an extended standing pressure $p_{standing, extended}$ with the user being in a standing position,
   - an extended static stiffness index ESSI, the extended static stiffness index being determined by subtracting the resting pressure $p_{rest}$ from the extended standing pressure $p_{standing, extended}$;
   - a difference $ESSI_1 - ESSI_2$ between at least two extended static stiffness indices $ESSI_1$ and $ESSI_2$, the extended static stiffness index $ESSI_1$ being determined by subtracting a first resting pressure $p_{rest1}$ from a first extended standing pressure $p_{standing, extended 1}$, the extended static stiffness index $ESSI_2$ being determined by subtracting a second resting pressure $p_{rest2}$ from a second extended standing pressure $p_{standing, extended 2}$;
   - a ratio $ESSI_1 : ESSI_2$ of at least two extended static stiffness indices $ESSI_1$ and $ESSI_2$, the extended static stiffness index $ESSI_1$ being determined by subtracting a first resting pressure $p_{rest1}$ from a first extended standing pressure $p_{standing, extended 1}$, the extended static stiffness index $ESSI_2$ being determined by subtracting a second resting pressure $p_{rest2}$ from a second extended standing pressure $p_{standing, extended 2}$;
   - a difference between at least two extended standing pressures $p_{standing, extended 1}$ and $p_{standing, extended 2}$ acquired at at least two different points in time;
   - a ratio of at least two extended standing pressures $p_{standing, extended 1}$ and $p_{standing, extended 2}$ acquired at at least two different points in time.

2. The monitoring system (116) according to the preceding claim, wherein the monitoring system (116) further comprises at least one display and control device (124), wherein the at least one display and control device (124) is adapted to communicate with the at least one measuring device (120).

3. The monitoring system (116) according to the preceding claim, wherein the at least one display and control device (124) is a mobile communication device, preferably a smartphone.

4. The monitoring system (116) according to one of the preceding claims, wherein the at least one measuring device (120) is adapted to be integrated into the compression device (112) and/or attached to the compression device (112), preferably on an outer side of the compression device (112).

5. The monitoring system (116) according to one of the preceding claims, wherein the at least one evaluation unit (126) is adapted to automatically determine if the user is sleeping and to switch into a sleep mode.

6. The monitoring system (116) according to one of the preceding claims, wherein the at least one evaluation unit (126) is adapted to determine if the user is walking.

7. The monitoring system (116) according to one of the preceding claims, wherein the at least one attitude sensor (122) comprises at least one orientation sensor, wherein the at least one orientation sensor comprises at least one of a gyroscope, an inclinometer, an altimeter, a magnetic field sensor, an angulation sensor and a tilt sensor.

8. The monitoring system (116) according to one of the two preceding claims, wherein the at least one evaluation unit (126) is adapted to generate a warning output via the at least one indicator device in case one or more of the following situations are recognized:

   - the compression device (112) is found to be ineffective;
   - the compression device (112) is found to exert an overpressure;
   - an external overpressure is found to act onto the compression device (112).

9. The monitoring system (116) according to one of the preceding claims, wherein the monitoring system (116) further comprises at least one foot pressure sensor, wherein the at least one foot pressure sensor is adapted to be positioned underneath at least one foot of the user and to acquire at least one force exerted by a weight of the user.

10. The monitoring system (116) according to one of the preceding claims, wherein the monitoring system (116) further comprises at least one motion sensor, wherein the at least one motion sensor is adapted to acquire at least one information regarding a motion of the user or a part of the user.

11. The monitoring system (116) according to one of the preceding claims, wherein the at least one evaluation unit (126) is adapted to compare the key figure K to at least one efficacy threshold for automatically determining the efficacy of the compression device (112).

12. The monitoring system (116) according to one of the preceding claims, wherein the at least one evaluation unit (126) is adapted to determine at least two different key figures $K_1$ and $K_2$, wherein the evaluation unit (126) is adapted to automatically determine the efficacy of the compression device (112) by a combination of the at least two key figures $K_1$ and $K_2$.

## Patentansprüche

1. Ein Überwachungssystem (116) zum Bestimmen der Wirksamkeit mindestens einer Kompressionsvorrichtung (112) zur Verwendung in einer Kompressionstherapie, wobei das Überwachungssystem (116) umfasst:

   - mindestens einen Drucksensor (118) zum Messen eines Drucks, der von der Kompressionsvorrichtung (112) auf einen Körperteil eines Benutzers ausgeübt wird;
   - mindestens einen Haltungssensor (122) zum Erfassen mindestens einer Haltungsinformation zu mindestens einer von einer Position, einer Ausrichtung und einer Bewegung des Benutzers;
   - mindestens eine Messvorrichtung (120) mit mindestens einer Auswertungseinheit (126), wobei die Messvorrichtung (120) dafür angepasst ist, um mit dem mindestens einen Drucksensor (118) und dem mindestens einen Haltungssensor (122) zu kommunizieren, wobei die mindestens eine Auswertungseinheit (126) dafür angepasst ist, um mindestens einen Druckwert zu empfangen, der von dem mindestens einen Drucksensor (118) erfasst wird, und wobei die mindestens eine Auswertungseinheit (126) dafür angepasst ist, um mindestens eine Haltungsinformation zu empfangen, die von dem mindestens einen Haltungssensor (122) erfasst wird;

      wobei die mindestens eine Auswertungseinheit (126) dafür angepasst ist, um den mindestens einen Druckwert und die mindestens eine Haltungsinformation automatisch zu kombinieren, um mindestens eine Kennzahl K zu bestimmen, die die Wirksamkeit der Kompressionsvorrichtung (112) unter Berücksichtigung der mindestens einen Haltungsinformation angibt, wobei die mindestens eine Auswertungseinheit (126) dafür angepasst ist, um eine Echtzeitbestimmung der Kennzahl auszuführen; und

**dadurch gekennzeichnet, dass** die Auswertungseinheit (126) ferner dafür angepasst ist, um den folgenden Vorgang zum Bestimmen eines Drucks bei längerem Stehen $p_{längeres\ Stehen}$ auszuführen:

Erfassen einer Messkurve von Druckwerten, nach einem Positionswechsel des Benutzers in die stehende Position; Automatisches Vergleichen einer Steigung der Messkurve mit mindestens einem Endpunkt-Schwellenwert, und, abhängig von einem Ergebnis des Vergleichs, automatisches Erkennen eines Endpunkts einer durch den Positionswechsel induzierten Änderung der Messkurve, und Zuweisen eines Druckwerts, der bei oder nach dem Endpunkt erfasst wird, dem Druck bei längerem Stehen $p_{längeres\ Stehen}$; und dadurch, dass die mindestens eine Kennzahl ausgewählt ist aus der Gruppe bestehend aus:

- einem Druck bei längerem Stehen $p_{längeres}$ Stehen, wobei der Benutzer eine stehende Position einnimmt,
- einem Index für die anhaltende statische Steifigkeit ESSI, wobei der Index für die anhaltende statische Steifigkeit durch Subtrahieren des Ruhedrucks $p_{rest}$ vom Druck bei längerem Stehen $p_{längeres\ Stehen}$ bestimmt wird;
- einer Differenz $ESSI_1 - ESSI_2$ zwischen mindestens zwei Indizes für die anhaltende statische Stei-figkeit $ESSI_1$ und $ESSI_2$, wobei der Index für die anhaltende statische Steifigkeit $ESSI_1$ durch Subtra-hieren eines ersten Ruhedrucks $p_{rest1}$ von einem ersten Druck bei längerem Stehen $p_{längeres\ Stehen\ 1}$ bestimmt wird, wobei der Index für die anhaltende statische Steifigkeit $ESSI_2$ durch Subtrahieren eines zweiten Ruhedrucks $p_{rest2}$ von einem zweiten Druck bei längerem Stehen $p_{längeres\ Stehen\ 2}$ bestimmt wird;
- einem Verhältnis $ESSI_1 : ESSI_2$ von mindestens zwei Indizes für die anhaltende statische Steifigkeit $ESSI_1$ und $ESSI_2$, wobei der Index für die anhaltende statische Steifigkeit $ESSI_1$ durch Subtrahieren eines ersten Ruhedrucks $p_{rest1}$ von einem ersten Druck bei längerem Stehen $p_{längeres\ Stehen\ 1}$ bestimmt wird, wobei der Index für die anhaltende statische Steifigkeit $ESSI_2$ durch Subtrahieren eines zweiten Ruhedrucks $p_{rest2}$ von einem zweiten Druck bei längerem Stehen $p_{längeres\ Stehen\ 2}$ bestimmt wird;
- einer Differenz zwischen mindestens zwei Drücken bei längerem Stehen $p_{längeres\ Stehen\ 1}$ und $p_{längeres\ Stehen\ 2}$, die zu mindestens zwei verschiedenen Zeitpunkten erfasst werden;
- einem Verhältnis von mindestens zwei Drücken bei längerem Stehen $p_{längeres\ Stehen\ 1}$ und $p_{längeres\ Stehen\ 2}$, die zu mindestens zwei verschiedenen Zeitpunkten erfasst werden.

2. Das Überwachungssystem (116) nach dem vorstehenden Anspruch, wobei das Überwachungssystem (116) ferner mindestens eine Anzeige- und Steuervorrichtung (124) umfasst, wobei die mindestens eine Anzeige- und Steuervorrichtung (124) dafür angepasst ist, um mit der mindestens einen Messvorrichtung (120) zu kommunizieren.

3. Das Überwachungssystem (116) nach dem vorstehenden Anspruch, wobei die mindestens eine Anzeige- und Steuervorrichtung (124) eine mobile Kommunikationsvorrichtung, vorzugsweise ein Smartphone, ist.

4. Das Überwachungssystem (116) nach einem der vorstehenden Ansprüche, wobei die mindestens eine Messvorrichtung (120) dafür angepasst ist, um in die Kompressionsvorrichtung (112) integriert und/oder an der Kompressionsvorrichtung (112) angebracht zu werden, vorzugsweise auf einer Außenseite der Kompressionsvorrichtung (112).

5. Das Überwachungssystem (116) nach einem der vorstehenden Ansprüche, wobei die mindestens eine Auswertungseinheit (126) dafür angepasst ist, um automatisch zu bestimmen, ob der Benutzer schläft und in einen Schlafmodus umzuschalten.

6. Das Überwachungssystem (116) nach einem der vorstehenden Ansprüche, wobei die mindestens eine Auswertungseinheit (126) dafür angepasst ist, um zu bestimmen, ob der Benutzer geht.

7. Das Überwachungssystem (116) nach einem der vorstehenden Ansprüche, wobei der mindestens eine Haltungssensor (122) mindestens einen Ausrichtungssensor umfasst, wobei der mindestens eine Ausrichtungssensor mindestens eines von einem Gyroskop, einem Neigungsmesser, einem Höhenmesser, einem Magnetfeldsensor, einem Angulierungssensor und einem Neigungssensor umfasst.

8. Das Überwachungssystem (116) nach einem der beiden vorstehenden Ansprüche, wobei die mindestens eine Auswertungseinheit (126) dafür angepasst ist, um eine Warnausgabe über die mindestens eine Anzeigevorrichtung zu erzeugen, falls eine oder mehrere der folgenden Situationen erkannt werden:

- es wird festgestellt, dass die Kompressionsvorrichtung (112) unwirksam ist;
- es wird festgestellt, dass die Kompressionsvorrichtung (112) einen Überdruck ausübt;
- es wird festgestellt, dass ein externer Überdruck auf die Kompressionsvorrichtung (112) wirkt.

9. Das Überwachungssystem (116) nach einem der vorstehenden Ansprüche, wobei das Überwachungssystem (116) ferner mindestens einen Fußdrucksensor umfasst, wobei der mindestens eine Fußdrucksensor dafür angepasst ist, um unter mindestens einem Fuß des Benutzers positioniert zu werden, und um mindestens eine Kraft zu erfassen, die von einem Gewicht des Benutzers ausgeübt wird.

10. Das Überwachungssystem (116) nach einem der vorstehenden Ansprüche, wobei das Überwachungssystem (116) ferner mindestens einen Bewegungssensor umfasst, wobei der mindestens eine Bewegungssensor dafür angepasst ist, um mindestens eine Information bezüglich einer Bewegung des Benutzers oder eines Teils des Benutzers zu erfassen.

11. Das Überwachungssystem (116) nach einem der vorstehenden Ansprüche, wobei die mindestens eine Auswertungseinheit (126) dafür angepasst ist, um die Kennzahl K mit mindestens einem Wirksamkeitsschwellenwert zum automatischen Bestimmen der Wirksamkeit der Kompressionsvorrichtung (112) zu vergleichen.

12. Das Überwachungssystem (116) nach einem der vorstehenden Ansprüche, wobei die mindestens eine Auswertungseinheit (126) dafür angepasst ist, um mindestens zwei unterschiedliche Kennzahlen $K_1$ und $K_2$ zu bestimmen, wobei die Auswertungseinheit (126) dafür angepasst ist, um die Wirksamkeit der Kompressionsvorrichtung (112) durch eine Kombination der mindestens zwei Kennzahlen $K_1$ und $K_2$ automatisch zu bestimmen.

**Revendications**

1. Système de surveillance (116) pour déterminer l'efficacité d'au moins un dispositif de compression (112) pour utilisation dans une thérapie de compression, le système de surveillance (116) comprenant :

- au moins un capteur de pression (118) pour mesurer une pression exercée sur une partie de corps d'un utilisateur par le dispositif de compression (112) ;
- au moins un capteur d'attitude (122) pour acquérir au moins une information d'attitude concernant au moins un parmi une position, une orientation et un mouvement de l'utilisateur ;
- au moins un dispositif de mesure (120) ayant au moins une unité d'évaluation (126), dans lequel le dispositif de mesure (120) est conçu pour communiquer avec l'au moins un capteur de pression (118) et l'au moins un capteur d'attitude (122), dans lequel l'au moins une unité d'évaluation (126) est conçue pour recevoir au moins une valeur de pression acquise par l'au moins un capteur de pression (118) et dans lequel l'au moins une unité d'évaluation (126) est conçue pour recevoir au moins une information d'attitude acquise par l'au moins un capteur d'attitude (122) ;

dans lequel l'au moins une unité d'évaluation (126) est conçue pour combiner automatiquement l'au moins une valeur de pression et l'au moins une information d'attitude afin de déterminer au moins un chiffre clé K indiquant l'efficacité du dispositif de compression (112) en tenant compte de l'au moins une information d'attitude, dans lequel l'au moins une unité d'évaluation (126) est conçue pour mettre en œuvre une détermination en temps réel du chiffre clé ; et
**caractérisé en ce que** l'unité d'évaluation (126) est conçue en outre pour mettre en œuvre la procédure suivante pour déterminer une pression debout en extension $p_{standing, extended}$ :
l'acquisition d'une courbe de mesure de valeurs de pression après un changement de position de l'utilisateur dans la position debout ; la comparaison automatique d'une pente de la courbe de mesure à au moins une valeur seuil de point d'extrémité, en fonction d'un résultat de la comparaison, la détection automatique d'un point d'extrémité d'un changement dans la courbe de mesure induit par le changement de position, et l'attribution d'une valeur de pression acquise au niveau du ou après le point d'extrémité à la pression debout en extension $p_{standing\ extended}$ ; et **en ce que** l'au moins un chiffre clé est choisi dans le groupe constitué de :

  ▪ une pression debout en extension $p_{standing,\ extended}$ avec l'utilisateur se trouvant en position debout,

■ un indice de rigidité statique en extension ESSI, l'indice de rigidité statique en extension étant déterminé en soustrayant la pression au repos $p_{rest}$ de la pression debout en extension $p_{standing, extended}$ ;

■ une différence $ESSI_1 - ESSI_2$ entre au moins deux indices de rigidité statique en extension $ESSI_1$ et $ESSI_2$, l'indice de rigidité statique en extension $ESSI_1$ étant déterminé en soustrayant une première pression au repos $p_{rest1}$ d'une première pression debout en extension $p_{standing, extended 1}$, l'indice de rigidité statique en extension $ESSI_2$ étant déterminé en soustrayant une deuxième pression au repos $p_{rest2}$ d'une deuxième pression debout en extension $p_{standing, extended 2}$ ;

■ un rapport $ESSI_1 : ESSI_2$ d'au moins deux indices de rigidité statique en extension $ESSI_1$ et $ESSI_2$, l'indice de rigidité statique en extension $ESSI_1$ étant déterminé en soustrayant une première pression au repos $p_{rest1}$ d'une première pression debout en extension $p_{standing, extended 1}$, l'indice de rigidité statique en extension $ESSI_2$ étant déterminé en soustrayant une deuxième pression au repos $p_{rest2}$ d'une deuxième pression debout en extension $p_{standing, extended 2}$ ;

■ une différence entre au moins deux pressions debout en extension $P_{standing, extended 1}$ et $P_{standing, extended 2}$ acquises au niveau d'au moins deux points différents dans le temps ;

■ un rapport d'au moins deux pressions debout en extension $p_{standing, extended 1}$ et $P_{standing, extended 2}$ acquises au niveau d'au moins deux points différents dans le temps.

2. Système de surveillance (116) selon la revendication précédente, dans lequel le système de surveillance (116) comprend en outre au moins un dispositif d'affichage et de commande (124), dans lequel l'au moins un dispositif d'affichage et de commande (124) est conçu pour communiquer avec l'au moins un dispositif de mesure (120).

3. Système de surveillance (116) selon la revendication précédente, dans lequel l'au moins un dispositif d'affichage et de commande (124) est un dispositif de communication mobile, de préférence un téléphone intelligent.

4. Système de surveillance (116) selon l'une des revendications précédentes, dans lequel l'au moins un dispositif de mesure (120) est conçu pour être intégré dans le dispositif de compression (112) et/ou fixé au dispositif de compression (112), de préférence sur un côté externe du dispositif de compression (112).

5. Système de surveillance (116) selon l'une des revendications précédentes, dans lequel l'au moins une unité d'évaluation (126) est conçue pour déterminer automatiquement si l'utilisateur est en train de dormir et pour commuter dans un mode sommeil.

6. Système de surveillance (116) selon l'une des revendications précédentes, dans lequel l'au moins une unité d'évaluation (126) est conçue pour déterminer si l'utilisateur est en train de marcher.

7. Système de surveillance (116) selon l'une des revendications précédentes, dans lequel l'au moins un capteur d'attitude (122) comprend au moins un capteur d'orientation, dans lequel l'au moins un capteur d'orientation comprend au moins un d'un gyroscope, d'un inclinomètre, d'un altimètre, d'un capteur de champ magnétique, d'un capteur d'angulation et d'un capteur d'inclinaison.

8. Système de surveillance (116) selon l'une des deux revendications précédentes, dans lequel l'au moins une unité d'évaluation (126) est conçue pour générer une sortie d'avertissement par l'intermédiaire de l'au moins un dispositif indicateur dans un cas où une ou plusieurs des situations suivantes sont reconnues :

- il s'avère que le dispositif de compression (112) est inefficace ;
- il s'avère que le dispositif de compression (112) exerce une surpression ;
- il s'avère qu'une surpression externe agit sur le dispositif de compression (112).

9. Système de surveillance (116) selon l'une des revendications précédentes, dans lequel le système de surveillance (116) comprend en outre au moins un capteur de pression de pied, dans lequel l'au moins un capteur de pression de pied est conçu pour être positionné en dessous d'au moins un pied de l'utilisateur et pour acquérir au moins une force exercée par un poids de l'utilisateur.

10. Système de surveillance (116) selon l'une des revendications précédentes, dans lequel le système de surveillance (116) comprend en outre au moins un capteur de mouvement, dans lequel l'au moins un capteur de mouvement est conçu pour acquérir au moins une information concernant un mouvement de l'utilisateur ou d'une partie de l'utilisateur.

**11.** Système de surveillance (116) selon l'une des revendications précédentes, dans lequel l'au moins une unité d'évaluation (126) est conçue pour comparer le chiffre clé K à au moins un seuil d'efficacité pour déterminer automatiquement l'efficacité du dispositif de compression (112).

**12.** Système de surveillance (116) selon l'une des revendications précédentes, dans lequel l'au moins une unité d'évaluation (126) est conçue pour déterminer au moins deux chiffres clés différents $K_1$ et $K_2$, dans lequel l'unité d'évaluation (126) est conçue pour déterminer automatiquement l'efficacité du dispositif de compression (112) par une combinaison des au moins deux chiffres clés $K_1$ et $K_2$.

*FIG. 1A*

*FIG. 1B*

*FIG. 1C*

*FIG. 2*

FIG. 3

FIG. 4

FIG. 5

FIG. 6

*FIG. 7*

*FIG. 8*

FIG. 9

FIG. 10

FIG. 11

**REFERENCES CITED IN THE DESCRIPTION**

**Patent documents cited in the description**

- US 2009055148 A
- WO 2008003920 A1
- US 20100010405 A1
- US 20110015498 A1
- US 20120083712 A1
- US 6231532 B1
- US 7127370 B2

**Non-patent literature cited in the description**

- **H. PARTSCH et al.** Measurement of lower leg compression in vivo: Recommendations for the performance of measurements of interface pressure and stiffness: A consensus statement. *Dermatol Surg,* 2006, vol. 32, 224-233
- **G. MOSTI et al.** Comparison between a new, two-component compression system with zinc paste bandages for leg ulcer healing: a prospective, multicenter, randomized, controlled trial monitoring sub-bandage pressures. *Wounds,* 2011, vol. 23 (5), 126-134